# EUROPEAN PATENT APPLICATION

(11) **EP 1 441 030 A1**
(43) Date of publication of application: **28.07.2004**
(21) Application number: 02801543.6
(22) Date of filing: 11.10.2002
(51) Int. Cl.: C12N 15/09, C07K 19/00, C07K 1/13, C07K 1/22, C12P 21/00, G01N 33/53

(54) **METHOD OF PURIFYING RECOMBINANT FUSED PROTEIN AND METHOD OF PRODUCING PROTEIN USING THE SAME**

(30) Priority: 11.10.2001 JP 2001314079
(71) Applicant: Katakura Industries Co., Ltd., Tokyo 104-8312 (JP)
(72) Inventor: NOMURA, Tsuyoshi, Kawagoe-shi, Saitama 350-1162 (JP); NAGAYA, Hidekazu, Mitaka-shi, Tokyo 181-0001 (JP); SHIMAMURA, Akio, Koshigaya-shi, Saitama 343-0034 (JP); NAYA, Shinichi, Wako-shi, Saitama 351-0114 (JP); KANAYA, Toshimichi, Ina-shi, Nagano 396-0111 (JP); SAKKA, Kazuo, Tsu-shi, Mie 514-0061 (JP); OHMIYA, Kunio, Tsu-shi, Mie 514-0005 (JP)
(74) Representative: Hartz, Nikolai F., Dr.
(86) International application number: PCT/JP2002/010578
(87) International publication number: WO 2003/033695

(57) **Abstract**

A method of purifying a recombinant fused protein characterized in that a recombinant fused protein, wherein a target protein has been fused with dockerin by genetic engineering techniques, is treated with a support having a cohesin domain immobilized thereon; and a method of producing a target protein characterized by comprising obtaining a recombinant fused protein having the target protein bound to dockerin with the use of an expression vector having a gene encoding the target protein and the dockerin inserted thereinto, subsequently purifying the recombinant fused protein by binding to a support having a cohesin domain immobilized thereon, and then eliminating the dockerin from the recombinant fused protein are disclosed.

Using these methods, it becomes possible to provide an affinity purification technique with the use of specific binding whereby a high affinity and easy dissociation can be established without causing insolubilization or inactivation.

## Description

### Technical Field

The present invention relates to a method of purifying a recombinant fused protein and a method of producing a target protein using the same. More specifically, the present invention relates to a method of purifying a recombinant fused protein with the use of specific binding between cohesin and dockerin and a method of producing a target protein using the same.

### Background Art

As a method of purifying proteins, gel filtration separating proteins with different molecular sizes by using molecular sieving property of a cross-linked polymer gel, ion-exchange chromatography separating proteins with the use of electrostatic binding between proteins and dissociation groups and hydrophobic chromatography with the use of binding property of proteins to hydrophobic groups and the like have been used. These methods are based on the physical or chemical differences of protein molecules, and proteins can be separated and purified by these methods no relating to the specific affinity of the protein's function. Accordingly, when a protein is purified by these methods, several methods need to be combined.

In view of this, a method of separating and purifying proteins with the use of specific binding between, for example, a hormone and its receptor, or an antigen and an antibody has been developed recently. By the recent development of genetic engineering techniques, techniques of a separation method and a purification method with the use of these specific bindings have been improved. For example, a method of easily purifying a given protein is performed by preparing an expression vector having a nucleotide sequence encoding the given protein fused with a nucleotide sequence encoding a fragment of a protein with a high affinity for a certain ligand (hereinafter referred to as 'affinity peptide'), introducing the vector into a given host to have a recombinant protein expressed to obtain the recombinant protein fused with the affinity peptide, and using a support having the ligand for the fused affinity peptide immobilized thereon. At present, this method is known to be very useful in isolating a recombinant protein easily.

Specifically, amethod using polyhistidine as an affinity peptide has been disclosed in Japanese patent number 2,686,090, and a method using glutathione S-transferase (hereinafter referred to as 'GST') has been disclosed in JP-A-7-184663. In addition, it has been believed that only a given protein can be specifically eluted with leaving an affinity peptide bound to a ligand of a support by reacting with a specific reagent or an enzyme in a purification step with the support having a ligand bound thereto if only a specific chemical or enzymatic cleavage site is inserted between the affinity peptide and the given recombinant protein. In fact, a method based on this idea has been conducted by Itakura et al., (Itakura et al., Science, vol. 198, pp1056-1063, (1977)). This method is also known to be very useful.

Recently, tens of thousand kinds of nucleotide sequences, which had not been discovered until now, were known by genome information. However, it is not clear that what function the protein encoded by the gene has in a living cell only with the data of the nucleotide sequence. Therefore, it is necessary that a gene be translated to a protein by recombinant DNA techniques and its property be examined. In order to examine the properties of the expressed recombinant proteins, a large amount of isolated recombinant protein is needed. There exist tens of thousand kinds of unknown genes, and also tens of thousand kinds of recombinant proteins produced therefrom. Therefore, it takes an enormous amount of time to investigate purification methods for these one by one, and it is extremely difficult to obtain an enormous amount of tens of thousand kinds of isolated unknown recombinant proteins promptly.

For this reason, as mentioned above, it is very useful to develop a technique by which an affinity peptide is fused with a recombinant protein by genetic engineering to have it expressed in a form of a recombinant protein fused with it, and a method of preparing a support immobilized a ligand corresponding to the affinity peptide thereon and using it for affinity purification.

Among the recombinant proteins fused with affinity peptides by genetic engineering techniques, some recombinant proteins which should be expressed to be originally water-soluble became insoluble by the effect of the fused affinity peptide. Particularly, among the recombinant proteins fused with polyhistidine which has been used as an affinity peptide until now, some became insoluble in a certain host, and some were expressed as a recombinant protein lost the original activities. Also, since GST has a high molecular weight of about 26 kDa, similar problems occur with the recombinant proteins fused with GST.

Most important factors in the above-mentioned affinity purification techniques include a high affinity between the fused affinity peptide and the corresponding ligand and ease of dissociation under mild conditions.

As a candidate of an affinity peptide which can be used for affinity purification techniques, a functional domain of a protein and its ligand molecule have been also investigated. One of them is a calcium ion binding motif, however, there is no applicable one to affinity purification so far with respect to the binding strength between the calcium ion binding motif and its ligand molecule and the ease of dissociation under mild conditions. This motif has been used only for confirmation of expression of recombinant proteins so far, in other words, it has been used only as a peptide for labeling. In addition, it is difficult to achieve techniques for obtaining a large amount of protein for a ligand binding to a calcium ion binding motif complementarily. Therefore, a method of purifying a recombinant protein with the use of this motif has not been established.

As a method of detecting or quantifying an affinity purified recombinant protein as mentioned above, a method using an antibody especially binding to an affinity peptide included in a recombinant protein, and a method using a ligand especially binding to an affinity peptide are known. Specifically, in a case where an antibody especially binding to a recombinant protein is used, a method using a monoclonal antibody or a polyclonal antibody is known. In a case where an affinity peptide and a ligand are used, a method using polyhistidine and nickel-nitrilotriacetic acid (Ni-NTA), and a method using a calmodulin-binding peptide (CBP) and calmodulin are known. (Stofco-Hahn et al., FEES Lett., Vol. 302, pp274-278 (1992); Carr et al., J. Biol. Chem., Vol. 266, pp14188-14192 (1991); Studier et al., Methods Enzymol., Vol. 185, pp60-89 (1990); Lowenstein, Cell, Vol. 70, 431-442 (1992))

However, in a case where an antibody against a recombinant protein for detecting or quantifying the recombinant protein is used, the following problems have occurred. For example, in a case where a polyclonal antibody is used as an antibody, a large amount of affinity peptide as an antigen is needed to produce the antibody. Also, it is difficult to prepare an anti-affinity peptide polyclonal antibody of uniform quality constantly because of individual differences of animals used for producing the antibody in the production yield or the characteristics of antibodies. In a case where a monoclonal antibody is used as an antibody, a hybridoma which produces a monoclonal antibody of uniform quality should be selected and maintained, which needs techniques, experiences and several months. And it is difficult to stably supply the monoclonal antibody over the long term.

In addition, in a case where a ligand with a high affinity to an affinity peptide is used for detecting or quantifying a recombinant protein, a target protein expressed in a form of a recombinant protein wherein the target protein has been fused with an affinity peptide may become insoluble, therefore, the target protein cannot be sometimes detected or quantified practically.

Accordingly, in an affinity purification technique with the use of specific binding, development of a technique by which a high affinity and easy dissociation of the binding is achieved without causing insolubilization or inactivation, and a technique by which easy detection and quantification of the target protein after affinity purification is achieved has been awaited.

### Disclosure of the invention

The present inventors proceeded with a variety of studies to solve the foregoing problems, and focused attention on a protein called cohesin, which has a calcium binding motif and is a part of the structure of cellulose binding protein called CipA derived from Clostridium josui (hereinafter referred to as 'C. josui'), which has been reported by Ohmiya et al., (Ohmiya et al., Miedai Seibutsushigen Kiyo, vol. 19, pp71-96 (1997)), and a protein called dockerin which binds complementarily to the cohesin via calcium ion.

As a result, it was found that a target protein can be isolated or detected easily by expressing the target protein in a form of a recombinant fused protein wherein the target protein has been fused with dockerin, and by using a cohesin domain as its specific ligand. The present invention has been thus worked out.

The present invention provides a method of purifying a recombinant fused protein characterized in that a recombinant fused protein, wherein a target protein has been fused with dockerin, is treated with a support having a cohesin domain immobilized thereon.

The present invention further provides a method of producing a target protein characterized by obtaining a recombinant fused protein having the target protein bound to dockerin with the use of an expression vector having genes encoding the target protein and the dockerin inserted thereinto, subsequently purifying the recombinant fused protein by binding to a support having a cohesin domain immobilized thereon, and then eliminating the dockerin from the recombinant fused protein.

The present invention further provides a method of detecting and quantifying a recombinant fused protein characterized by binding a recombinant fused protein having a target protein fused with dockerin to a labeled cohesin domain, and then measuring the labeled amount after eliminating the non-binding labeled cohesin domain from the measurement system.

The present invention further provides a method of detecting and quantifying a cohesin domain characterized by binding a cohesin domain to a labeled dockerin, and then measuring the labeled amount after eliminating the non-binding labeled dockerin.

The present invention further provides a method of purifying a cohesin domain characterized in that a solution containing a cohesin domain is treated by adsorption with an anion exchange support to have the cohesin domain adsorbed to the anion exchange support, and subsequently the cohesin domain is eluted by increasing salt concentration.

### Brief Description of the Drawings

Fig 1 is a drawing to show the flow of the construction of pYNG/Cj.
Fig 2 is a drawing to show detection of cohesin expressed by Cj1 recombinant virus and Cj2 recombinant virus by a cellulose binding assay.
(A) Cohesin expressed by Cj1 recombinant virus

| Description of reference numerals and signs M; Marker | |
|---|---|
| 1; Clone No.1 | 5; Clone No.6 |
| 2; Clone No.2 | 6; Clone No.7 |
| 3; Clone No.3 (selected clone) | 7; Blank |
| 4; Clone No.4 | 8; CJ2 Clone No.1 |

(B) Cohesin expressed by Cj2 recombinant virus

| Description of reference numerals and signs M; Marker | |
|---|---|
| 1; Clone No.1 | 5; Clone No.5 |
| 2; Clone No.2 | 6; Clone No.6 |
| 3; Clone No.3 (selected clone) | 7; Blank |
| 4; Clone No.4 | 8; Blank |

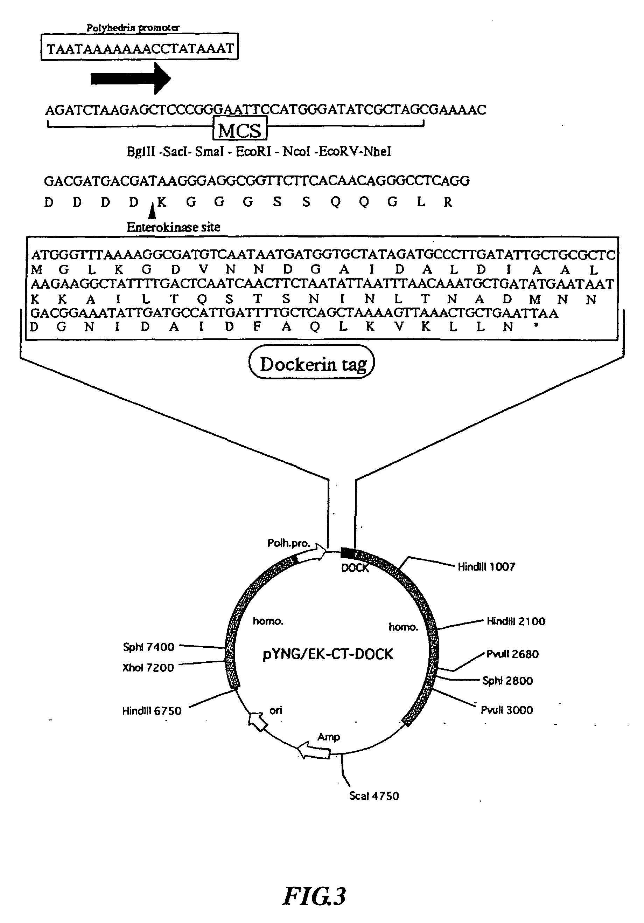

Fig. 3 is a drawing to show the structure of a vector for addition of a C-terminal dockerin (pYNG/EK-CT-DOCK)

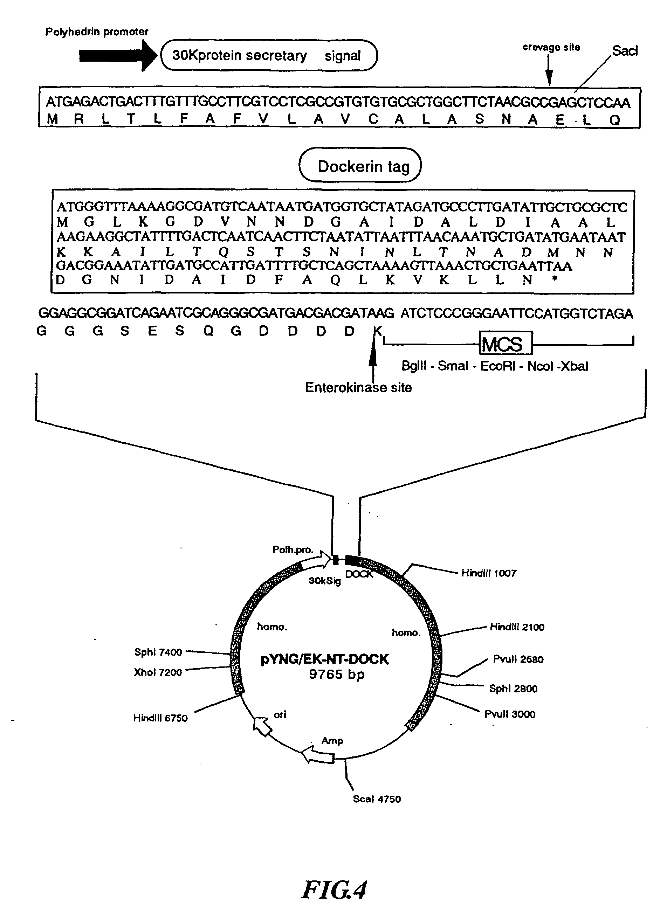

Fig. 4 is a drawing to show the structure of a vector for addition of an N-terminal dockerin (pYNG/EK-NT-DOCK)

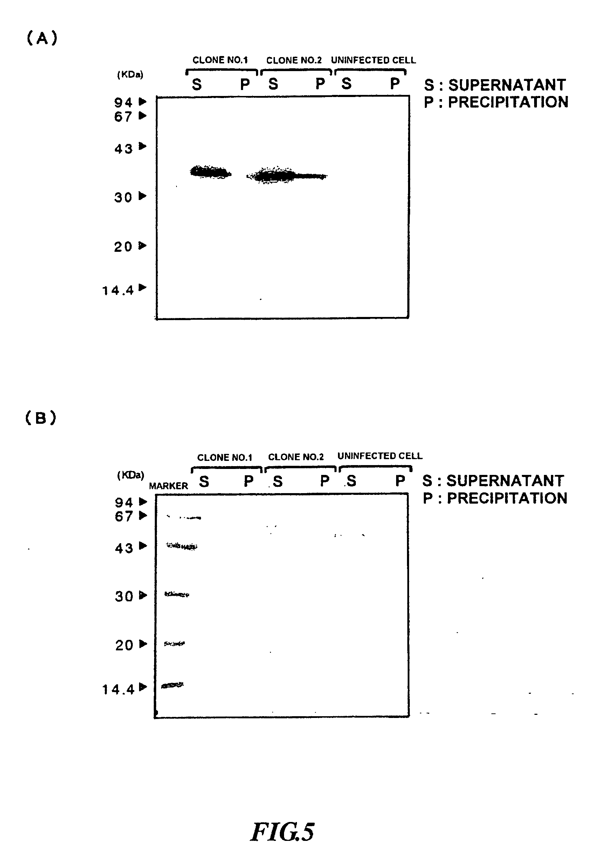

Fig. 5 is a drawing to show SDS-PAGE of cultured cells infected with a recombinant virus for expressing GFP fused with a C-terminal dockerin. (A) shows Western blotting using an anti-GFP antibody and (B) shows CBB staining.

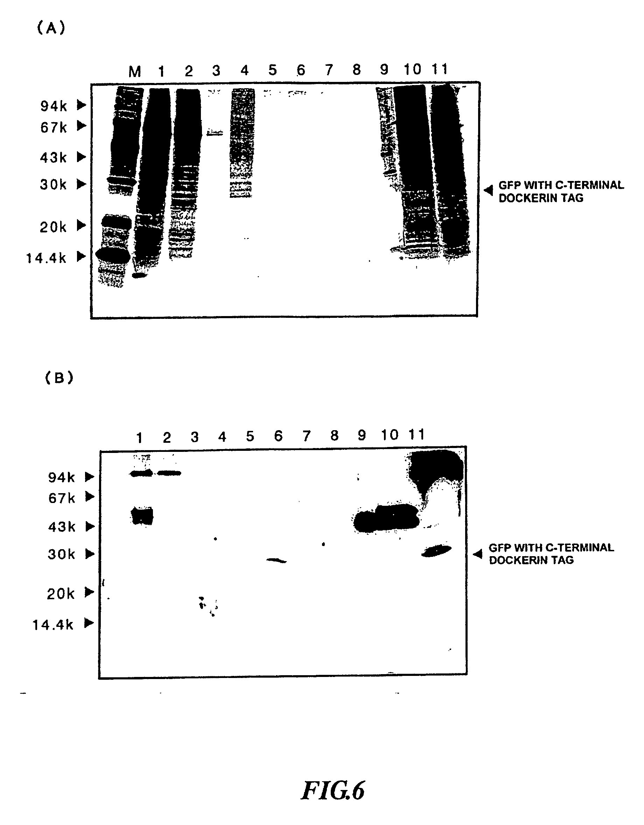

Fig. 6 is a drawing to show purification of GFP fused with a C-terminal dockerin from CBinD 100 Resin having cohesin bound thereto. (A) shows silver staining and (B) shows Western blotting using an anti-GFP antibody.
Description of reference numerals and signs
1: Supernatant of the culture medium that cohesin has been expressed
2: Remaining fraction 1 after binding the supernatant of the above-mentioned 1 to CBinD 100 Resin
3: Remaining fraction 2 after binding the supernatant of the above-mentioned 1 to CBinD 100 Resin
4: Remaining fraction 1 after applying the supernatant of the culture medium that GFP fused with a C-terminal dockerin has been expressed to cohesin binding CBinD 100 Resin
5: Remaining fraction 2 after applying the supernatant of the culture medium that GFP fused with a C-terminal dockerin has been expressed to the cohesin binding CBinD 100 Resin
6: Fraction 1 after eluting GFP fused with a C-terminal dockerin with a buffer containing EDTA
7: Fraction 2 after eluting GFP fused with a C-terminal dockerin with a buffer containing EDTA
8: Fraction 3 after eluting GFP fused with a C-terminal dockerin with a buffer containing EDTA
9: Fraction after eliminating cohesin domain from the cohesin binding CBinD 100 Resin with ethylene glycol.
10: CBinD 100 Resin after treatment of the above-mentioned 9
11: Supernatant of the culture medium that GFP fused with a C-terminal dockerin has been expressed

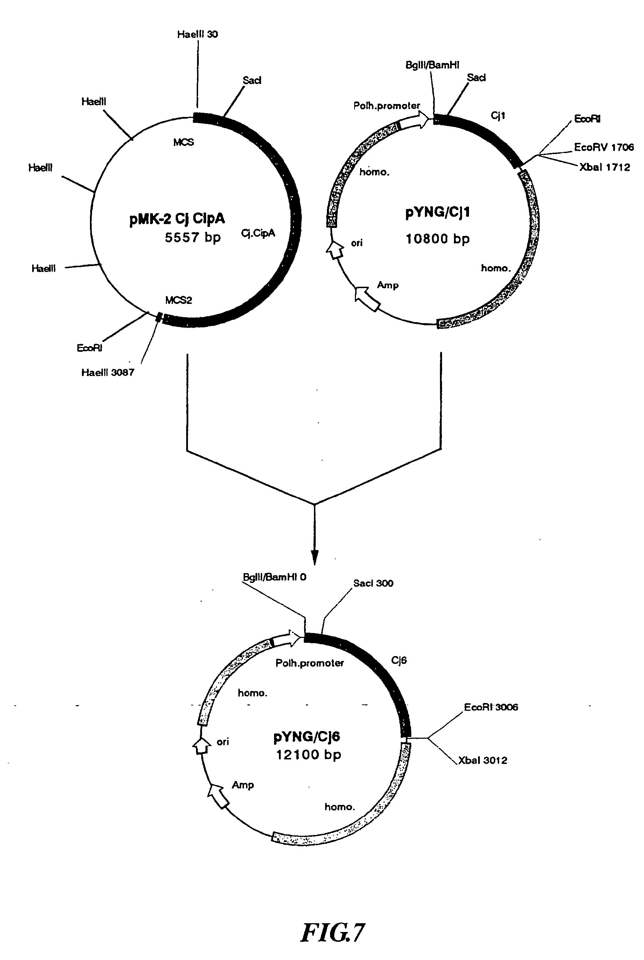

Fig. 7 is a drawing to show the flow of the construction of pYNG/Cj6.

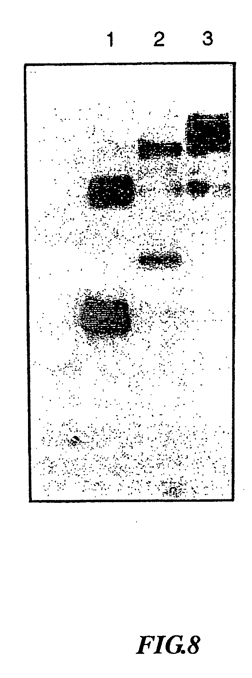

Fig. 8 is a drawing to show detection of a cohesin domain using GFP fused with a C-terminal dockerin
Description of reference numerals and signs
1: Detection of cohesin containing one cohesin domain using GFP fused with a C-terminal dockerin
2: Detection of cohesin containing two cohesin domains using GFP fused with a C-terminal dockerin
3: Detection of cohesin containing six cohesin domains using GFP fused with a C-terminal dockerin

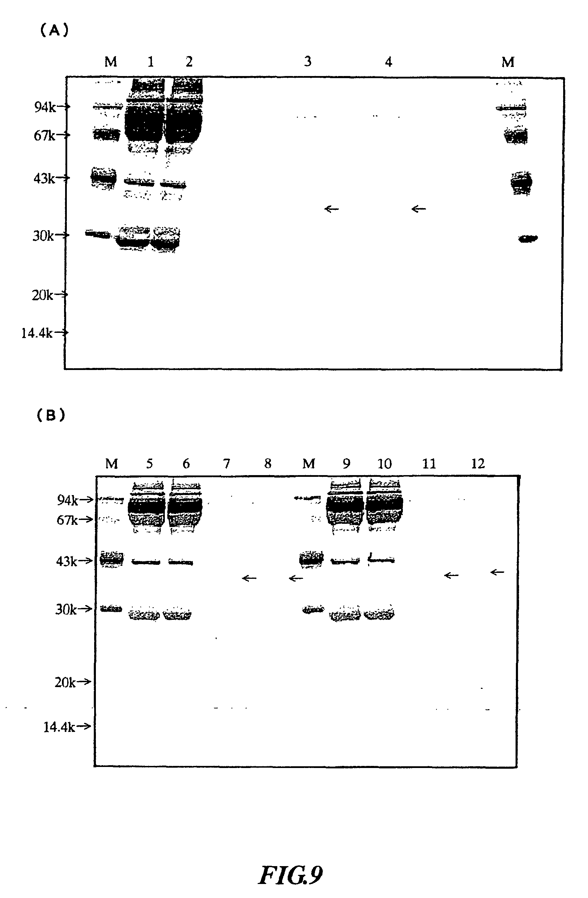

Figs. 9(A) and (B) are drawings to show purification of GFP fused with a C-terminal dockerin using CNBr-activated Sepharose 4 Fast Flow to which a cohesin protein (containing one, two or six cohesin domains) is covalently bound.
Description of reference numerals and signs
1: Body fluid of larvae in which GFP fusedwith a C-terminal dockerin has been expressed
2: The remaining fraction after bound to Cj1 resin
3: Fraction 1 of GFP fused with a C-terminal dockerin eluted from Cj1 resin with EDTA
4: Fraction 2 of GFP fused with a C-terminal dockerin eluted from Cj1 resin with EDTA
5: Body fluid of larvae in which GFP fused with a C-terminal dockerin has been expressed
6: Remaining fraction after bound to Cj2 resin
7: Fraction 1 of GFP fused with a C-terminal dockerin eluted from Cj2 resin with EDTA
8: Fraction 2 of GFP fused with a C-terminal dockerin eluted from Cj2 resin with EDTA
9: Body fluid of larvae in which GFP fused with a C-terminal dockerin has been expressed
10: Remaining fraction after bound to Cj6 resin
11: Fraction 1 of GFP fused with a C-terminal dockerin eluted from Cj6 resin with EDTA
12: Fraction 2 of GFP fused with a C-terminal dockerin eluted from Cj6 resin with EDTA
   M: Marker
   ←: GFP fused with a C-terminal dockerin

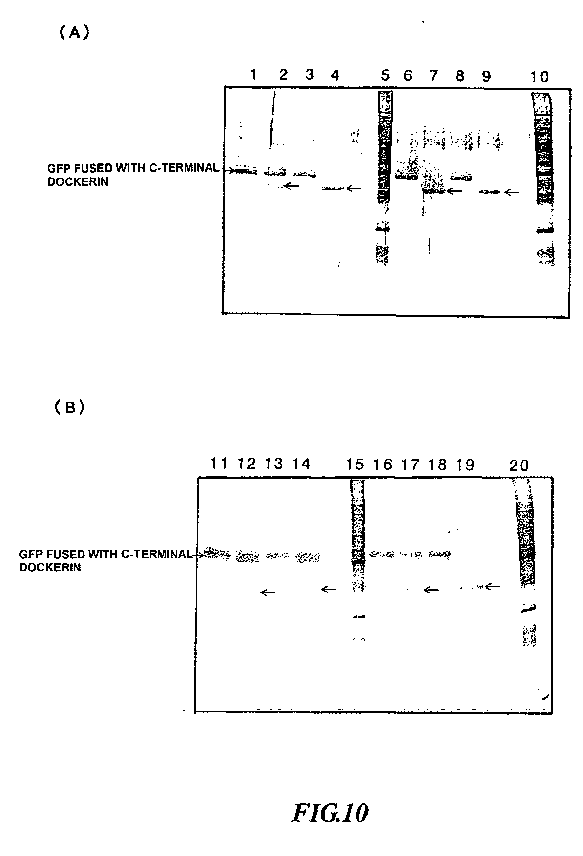

Figs. 10(A) and (B) are drawings to show purification of GFP fused with an N-terminal or C-terminal dockerin using CNBr-activated Sepharose 4 Fast Flow to which a cohesin is covalently bound and to show cleavage of tag with enterokinase.
Description of reference numerals and signs
1: Fraction 1-1 of GFP fused with a C-terminal dockerin eluted with EDTA
2: Fraction 1-1 cleaved by enterokinase at 4°C for 48 hours
3: Fraction 1-2 of GFP fused with a C-terminal dockerin eluted with EDTA
4: Fraction 1-2 cleaved by enterokinase at 4°C for 48 hours
5: resin after Fraction 1 was eluted
6: Fraction 2-1 of GFP fused with a C-terminal dockerin eluted with EGTA
7: Fraction 2-1 cleaved by enterokinase at 4°C for 48 hours
8: Fraction 2-2 of GFP fused with a C-terminal dockerin eluted with EGTA
9: Fraction 2-2 cleaved by enterokinase at 4°C for 48 hours
10: resin after Fraction 2 has been eluted
11: Fraction 3-1 of GFP fused with an N-terminal dockerin eluted with EDTA
12: Fraction 3-1 cleaved by enterokinase at 4°C for 48 hours
13: Fraction 3-2 of GFP fused with an N-terminal dockerin eluted with EDTA
14: Fraction 3-2 cleaved by enterokinase at 4°C for 48 hours
15: resin after Fraction 3 was eluted
16: Fraction 4-1 of GFP fused with an N-terminal dockerin eluted with EGTA
17: Fraction 4-1 cleaved by enterokinase at 4°C for 48 hours
18: Fraction 4-2 of GFP fused with an N-terminal dockerin eluted with EGTA
19: Fraction 4-2 cleaved with enterokinase at 4°C for 48 hours
20: resin after Fraction 4 was eluted
   ←: GFP after tag was cleaved

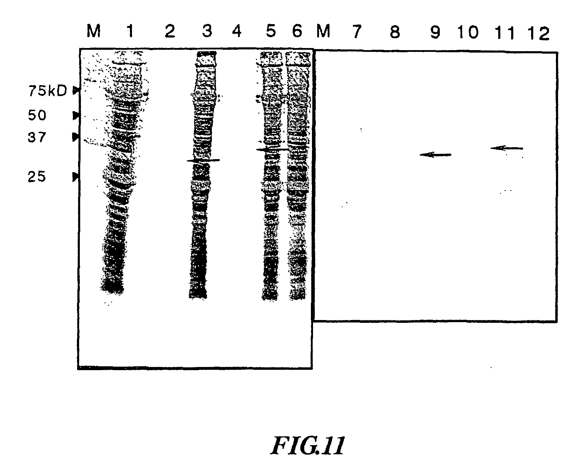

Fig. 11 is a drawing to show detection of a protein fused with dockerin by a biotinylated cohesin protein.
Description of reference numerals and signs
1: Homogenized pupae solution in which GFP fused with a C-terminal dockerin has been expressed
2: Purified product of GFP fused with a C-terminal dockerin
3: Homogenized pupae solution in which GFP fused with an N-terminal dockerin has been expressed
4: Purified product of GFP fused with an N-terminal dockerin
5: Homogenized pupae solution in which mouse interferon-beta fused with a C-terminal dockerin has been expressed
6: Homogenized pupae solution in which mouse interferon-beta fused with an N-terminal dockerin has been expressed
7: Homogenized pupae solution in which GFP fused with a C-terminal dockerin has been expressed
8: Purified product of GFP fused with a C-terminal dockerin has been expressed
9: Homogenized pupae solution in which GFP fused with an N-terminal dockerin has been expressed
10: Purified product of GFP fused with an N-terminal dockerin has been expressed
11: Homogenized pupae solution in which mouse interferon-beta fused with a C-terminal dockerin has been expressed
12: Homogenized pupae solution in which mouse interferon-beta fused with an N-terminal dockerin has been expressed
   ←: GFP fused with a dockerin

### Best Mode for Carrying Out the Invention

A recombinant fused protein wherein a target protein has been fused with dockerin used in the present invention can be obtained by inserting a gene encoding the target protein (hereinafter referred to as 'target protein gene') and a gene encoding dockerin (hereinafter referred to as 'dockerin gene') into an expression vector, and by introducing the expression vector into a given host to express the protein.

More specifically, the recombinant fused protein can be obtained by inserting a dockerin gene as an affinity peptide into the 5'-upstream region or the 3'-downstream region of a target protein gene, which is inserted into the downstream of the promoter of a given expression vector, thereby constructing an expression vector for producing the recombinant fused protein of the dockerin and the given protein, and then by introducing the expression vector into a host to produce the protein.

The dockerin genes used for constructing the above-mentioned expression vector include, for example, a gene cloned by a conventional method, which is from a cellulase complex derived from C. josui reported by Ohmiya et al. (Ohmiya et al., Miedai Seibutsushigen Kiyo, vol. 19, pp71-96 (1997)). Without being limited thereto, a dockerin gene reported by Ohmiya et al., (Ohmiya, Ket al., J. Bacteriology, vol. 180; pp4303-4308 (1998)) or by Fierobe et al., (Fierobe H. P et al., Biochemistry, vol. 38; pp12822-12832 (1999)) may be used. There is no particular restriction on the target protein genes, and therefore a gene encoding various known proteins may be used.

When the above-mentioned expression vector is constructed, a gene encoding a chemical or enzymatic cleavage site (hereinafter referred to as 'cleavage gene') may be inserted between the target protein region and the dockerin region of the recombinant fused protein in order to eliminate the dockerin easily. In a case where an enzymatic cleavage is used for a peptide sequence of the cleavage site, a peptide sequence which is recognized and cleaved by an adequate enzyme such as endoprotease and pro-hormone convertase may be used (Meth. Enzymol., vol. XIX, Perlman and Lorand edition, New York: Academic press (1970); Meth. Enzymol., vol. XLV, Lorand edition, New York: Academic press (1976)). More specific examples of the peptide sequence of cleavage site include a nucleotide sequence encoding an enterokinase cleavage site (DDDDK). In a case where a chemical cleavage is used for a peptide sequence of the cleavage site, a peptide sequence which is recognized and cleaved by a chemical reagent such as an organic acid and an inorganic acid, hydroxylamine, N-bromosuccinimide and cyanogenbromide may be used (Witcop, Advances in Protein Chemistry, Anfinseen et al. edition, pp231-321, New York: Academic Press (1961)). More specific examples of the peptide sequence of cleavage site include a nucleotide sequence encoding a cyanogenbromide cleavage site (MX; X is a given amino acid).

There is no particular restriction on the expression vectors into which the target protein gene and the dockerin gene, and if necessary, the cleavage gene are inserted. The expression vector may be selected depending on the host to be used. For example, in a case where *E*. *coli* bacteria is used as a host, pTRC99A (Amersham Biosciences) or the like is used. In a case where a cultured insect cell or an insect body is used as a host, it is preferable to use ABv (Katakura Industries Co., Ltd.) or the like.

A host for expressing the above-mentioned expression vector may be selected depending on the expression vector to be used. A recombinant fused protein can be expressed in the vector by a conventional method.

The obtained recombinant fused protein as mentioned above is treated with a support having a cohesin domain immobilized thereon (hereinafter referred to as 'immobilized cohesin support') and purified.

As a cohesin domain used in this step, any cohesin domains can be used as long as they bind especially to the above-mentioned dockerin. Preparation of the cohesin domain can be performed in accordance with the preparation of the recombinant fused protein. For example, the gene of the cohesin domain, which isfromCipAderivedfromC. josui, is cloned, the gene is inserted into a given expression vector to construct a vector expressing the cohesin domain, then the expression vector is introduced into a host, and thus the cohesin domain can be produced. When the cohesin domain is prepared, a gene encoding a binding site (hereinafter referred to as 'binding gene') for binding the cohesin domain to the support may, if necessary, be inserted into the vector. There is no particular restriction on the binding gene as long as it binds a cohesin domain to a support. For example, in a case where the above-mentioned CipA is used as a cohesin domain, a cellulose binding domain (CBD) of CipA may be used as a binding gene.

The thus obtained cohesin domain can be purified and isolated by chromatography with the use of an anion exchange resin. Specifically, a fraction containing the cohesin domain is adsorbed to an anion exchange resin by adsorptive treatment with an anion exchange support, subsequently the cohesin domain is eluted by increasing salt concentration, and thus the cohesin domain can be purified and isolated. The anion exchange support used for chromatography includes DEAE anion exchanger, QAE anion exchanger, Q ion exchanger and the like. As a specific brand name, Q-Sepharose anion exchanger (Amersham Biosciences) is given.

The purified cohesin domain can be detected by using a labeled dockerin, which will be described below. A green fluorescent protein (GFP) is used for labeling the dockerin. The recombinant fused protein of GFP and dockerin is produced by a conventional method, and the GFP can be detected with an anti-GFP antibody, or the fluorescence of GFP can be detected and quantified with a fluorescence detector.

Immobilization of the cohesin domain is performed by binding the cohesin domain to a support according to a conventional method. The cohesin domain can be directly bound to a support. However, a fused protein coupledwith, for example, a cellulose binding domain (CBD) or the like is produced by a conventional method, with which the cohesin domain can be bound to a support. In a case where a hydrogen bond is used as a bonding mechanism for binding a cohesin domain to a support, a cellulose support such as CBinD 100 Resin (Novagen) can be used as a support for immobilization when the above-mentioned fused protein in which a cellulose binding domain has been coupled with a cohesin domain is used because it can be adsorbed to the cellulose via the cellulose binding domain coupled with the cohesin domain. In a case where a covalent bond is used as a bonding mechanism for binding a cohesin domain to a support, an activated support such as CNBr-activated Sepharose 4 Fast Flow, CNBr-activated Sepharose 4B, EAH Sepharose 4B and ECH Sepharose 4B, and Epoxy-activated Sepharose 6B (Amersham Biosciences) can be used because the cohesin domain can be bound to the activated support with the use of a cross-linking agent or the like.

Examples of the specific methods of binding a recombinant fused protein to an immobilized cohesin support include, for example, a method of adding calcium ion to a sample containing a recombinant fused protein, then applying the mixture to an immobilized cohesin support. The support having a recombinant fused protein bound thereto as mentioned above is washed with an appropriate buffer, subsequently washed with an appropriate buffer containing a metal chelate such as EDTA, and thereby the recombinant fused protein is eluted.

This is because dockerin has 60 amino-acid residues (molecular weight; about 7 kDa), which is very small, and the dissociation constant of the dockerin and cohesin domain is 4.7×10⁻⁷, which is also very small. In addition, the dockerin is bound to the cohesin domain in the presence of calcium, and this complex has a property of dis sociating easily by eliminating calcium ion with a metal chelate.

Accordingly, the recombinant fused protein can be separated from other substances because the dockerin region is strongly bound to the immobilized cohesin support. In other words, the dockerin-cohesin complex is formed on the support by the above-mentioned strong binding, the support is thoroughly washed with an appropriate buffer to eliminate other substances, and then an appropriate buffer containing a metal chelate is applied to eliminate calcium that is needed for binding the dockerin region of the recombinant fused protein and the cohesin domain region of the solid phase. Thus, only the recombinant fused protein is eluted, and thereby it can be purified.

The buffers used in the purified step include Good buffer (Good, N.E. and Izawa, S, Method. Enzymol., vol. 24, Part B, pp53-68 (1972)): (hereinafter referred to as 'Good buffer'), phosphate buffer and the like. The metal chelates include ethylenediamine-N,N,N',N'-tetraacetic acid (hereinafter referred to as 'EDTA') and -ethylenedioxybis (ethylamine)-N,N,N',N'-tetraacetic acid (hereinafter referred to as 'EGTA') and the like.

Thus, the recombinant fused protein wherein the target protein has been fused with the dockerin is purified. The purified recombinant fused protein can be detected and quantified by using an antibody against a target protein or a labeled cohesin domain with the use of the binding property of a dockerin and a cohesin domain. In a case where an antibody is used for the detection and quantification, an antibody against a target protein or dockerin is produced and labeled according to a conventional method, and the resultant antibody may be used. In a case where a labeled cohesin domain is used, a cohesin domain prepared as mentioned above may be labeled according to a conventional method. As a label used for labeling the cohesin domain, the one which gives a signal to be detectable directly or indirectly is preferable, including enzymes, fluorescent proteins, radioactive labeled molecules, fluorescent agents, dyes, particles, chemiluminescent materials, enzyme substances or cofactors, enzyme inhibitors and magnetic particles and the like. Biotin is particularly preferable. The cohesin domain may be labeled with these labels according to a conventional method. Specifically, a biotinylated cohesin domain by using biotin as a label can be detected and quantified with the streptavidin, which has been labeled with alkaline phosphatase or horseradish peroxidase.

A method using a labeled cohesin domain is preferable to a method using an antibody against a target protein in that it can be applied regardless of the target protein.

The target protein can be obtained by eliminating the dockerin region from the purified recombinant fused protein as mentioned above.

The methods of eliminating the dockerin region from the recombinant fused protein include a method of cleaving a chemical or enzymatic cleavage site provided between the target protein region and the dockerin region of the recombinant fused protein. In other words, the recombinant fused protein, in which only the dockerin region can be easily eliminated, can be obtained by using an expression vector in which a gene encoding a chemical or enzymatic cleavage site has been inserted between the target protein gene and the dockerin gene in advance.

Accordingly, with respect to a recombinant protein, for example, only the target protein can be isolated and obtained by binding a recombinant fused protein to an immobilized cohesin support, subsequently washing it with an appropriate buffer such as Good buffer and phosphate buffer, then treating it with a specific reagent or enzyme, for example, in a case where the cleavage site is an enterokinase cleavage site, treating it with enterokinase to leave the dockerin bound to the cohesin domain of the immobilized cohesin support.

The present invention is based on the facts that dockerin has 60 amino-acid residues (molecular weight; about 7 kDa), which is very small, the dissociation constant of the dockerin and cohesin domain is 4.7×10⁻⁷, which is also very small, and the complex of dockerin and cohesin domain has a property of dissociating easily by eliminating calcium ion with a metal chelate.

More specifically, dockerin has a small molecular weight, and a recombinant fused protein wherein the target protein has been fused is unlikely to become insoluble. Also, the binding strength between the dockerin and the cohesin domain is strong enough to perform affinity purification. Furthermore, the dockerin and the cohesin domain binding can be separated easily by eliminating calcium ion with a metal chelate. These properties enable to provide a method of isolating a recombinant fused protein easily under mild purification conditions without making the target protein insoluble.

Since the dockerin and the cohesin domain, which are used in the above-mentioned method, can be easily obtained as a recombinant protein, it is possible to stably supply them as an affinity peptide and a ligand of uniform quality over a long term.

In a case where a chemical or enzymatic cleavage site is provided between the target protein and the dockerin of the recombinant fused protein, only the target protein can be obtained with a simple method.

Conventionally, in order to detect and quantify a target protein, an antibody against the target protein has to be prepared for each protein. If a recombinant fused protein containing dockerin and a target protein is used, the target protein can be detected and quantified easily with a labeled cohesin domain.

Furthermore, detection and quantification of a recombinant fused protein wherein a target protein has been fused with dockerin with a labeled cohesin domain according to the above-mentioned method will enable to measure or detect the expression amount of the target protein and localization or pharmacokinetics in the cells and tissues.

### Examples

The present invention will be illustrated in more detail with reference to the following examples, however, the present invention is not limited thereto.

### Example 1

### Acquisition of cohesin gene:

The cohesin gene was amplified by PCR under the conditions mentioned below by using pMK-2 Cj CipA vector (given by Associate Professor Sakka, Laboratory of Applied Microbiology, Faculty of Bioscience, Mie University), which was obtained by cloning the C. josui CipA gene (Ohmiya, K et al., J. Bacteriology, vol. 180; pp4303-4308 (1998)) into pMK vector, as a template.

### (1) Design of PCR primers

The nucleotide sequences and the combinations of primers used for the amplification of the cohesin gene are as follows.
Primer 1: for addition of a restriction enzyme BamHI site (for Cj1 or Cj2 amplification) 5'- ccggatccatgcgtaaaaagtctttagc -3'
Primer 2: for addition of a restriction enzyme XbaI site (for Cj1 amplification) 5'- ggtctagactaaggatctggacttataac -3'
Primer 3: for addition of a restriction enzyme XbaI site (for Cj2 amplification)

5'- ggtctagactaaggttctgggtcaccagg -3'

### (2) Preparation of PCR sample

| | |
|---|---|
| pMK/C. josui CipA | 100 pg |
| 10 x Ex-Taq buffer (Takara Shuzo Co., Ltd.) | 5 µl |
| Primer 1 | 1 ng |
| Primer 2 or Primer 3 | 1 ng |
| dNTP mix (Takara Shuzo Co., Ltd.) | 4 µl |
| Ex-Taq (Takara Shuzo Co., Ltd.) | 0.5 µl |
| Distilled water Added to make the total volume | 50 µl |

### (3) PCR conditions

Step 1: 94.0°C 2 minutes
Step 2: 94.0°C 30 seconds
Step 3: 55.0°C 45 seconds
Step 4: 72.0°C 60 seconds
Step 5: Repeat Steps 2 to 4 for 30 times
Step 6: 72.0°C 3 minutes

### Example 2

Construction of transfer vector for cohesin expression:

### (1) Preparation of cohesin gene

Restriction enzymes BamHI (8 to 24U) and XbaI (8 to 24U) (both from Takara Shuzo Co., Ltd.) were added to 20 µl of the C. josui CipA cohesin gene PCR product, which had a restriction enzyme BamHI recognition sequence at 5' end, one (Cj1) or two (Cj2) of cohesin domains and a restriction enzyme XbaI recognition sequence at 3' end. The restriction enzyme digestion was performed and the end sequences were exposed. Then, the restriction enzyme-treated cohesin gene fragment was purified by using a Qiagen spin column (QIAquick: Qiagen).

### (2) Insertion into transfer vector

After digesting the restriction enzyme recognition sites in the multi-cloning site of transfer vector pYNG for recombinant baculoviral preparation (Katakura Industries Co., Ltd.) with BglII and XbaI (both from Takara Shuzo Co., Ltd.), the digested ends were dephosphorylated with alkaline phosphatase (Takara Shuzo Co., Ltd.). Solution I (5 µl) of Ligation Kit (Takara Shuzo Co., Ltd.) was added to 0.1 µg (2 µl) of the linearized transfer vector and 0.1 µg (3 µl) of the restriction enzyme-treated cohesin gene fragment (containing one or two cohesin domains). After the reaction at 16°C for 1 hour, *E*. *coli* JM109 (Toyobo Co., Ltd.) was transformed with the whole reaction solution. Ampicillin resistant transformants were selected and plasmids were purified.

Then, the following primers were used to confirm the nucleotide sequence of several clones.

yng.for:

5'- aaccatctcgcaaataaata -3'

yng.rev:

5'- cgcacagaatctaacgctta -3'

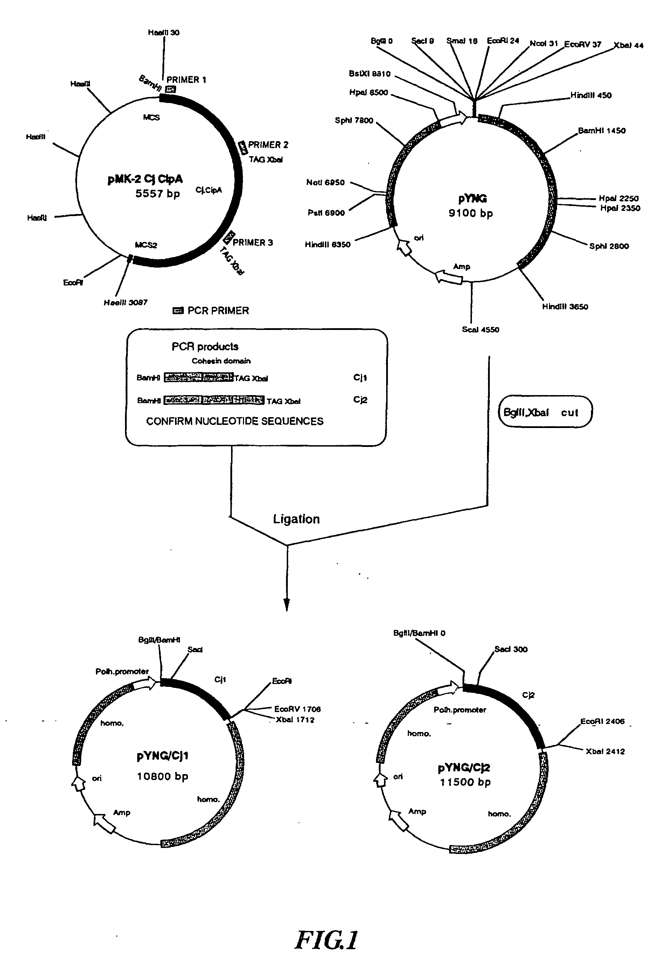

After the reaction with the above primers by using DNA Sequence Kit (Applied Biosystems Inc.), the analysis was performed by using an ABI Genetic Analyzer (Applied Biosystems Inc.). A clone harboring the cohesin gene fragment without mutations was selected and referred to as pYNG/Cj1 (containing one cohesin domain) or pYNG/Cj2 (containing two cohesin domains) (Fig. 1).

### Example 3

Preparation of cohesin-expressing recombinant virus (1):

### (1) Cotransfection

Firstly, using a 35 mm petri dish for cell culture, Bm-N cells were prepared in a confluent monolayer of about 1 x 10⁶ cells in static culture. The linearized ABv DNA (0.1 µg) (Katakura Industries Co., Ltd.) and either of 0.5 µg of the transfer vector pYNG/Cj1 or pYNG/Cj2 obtained as above were mixed with 100 µl of TC-100 (Nosan Corporation) in a 1.5 ml tube and left for 15 minutes at room temperature. The cationic lipid reagent (Lipofectin: GIBCO-BRL) (5 µl) was added to 100 µl of TC-100 and left for 15 minutes at room temperature. Then, after these two solutions were mixed and left for an additional 15 minutes at room temperature, 800 µl of TC-100 was added. This mixture was added to the Bm-N cells prepared in a confluent monolayer on the 35 mm petri dish for cell culture. After the cultivation for 20 hours at 25°C, the DNA solution was removed, 2 ml of TC-100 medium containing 10% FBS (Sigma) was added, then it was statically cultured for 7 days at 25°C. The supernatant of this culture medium was used as a recombinant virus stock solution.

### (2) Screening

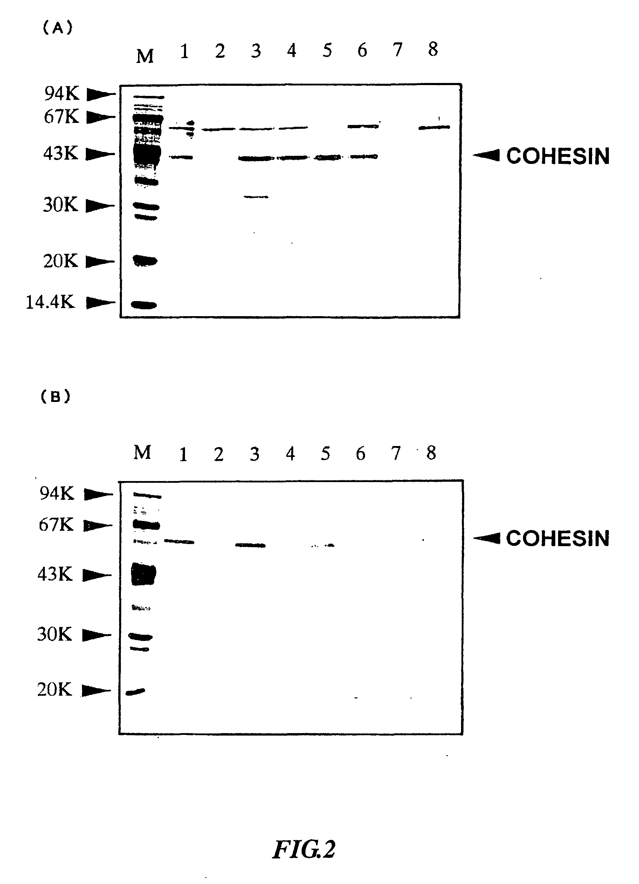

An isolation of ABv from the obtained virus stock solution as above was performed according to the method reported by King et al. (King, L A and Possee, R. D., A Laboratory Guide London: Chapman and Hall (1992)). First, the Bm-N cells were adjusted to 1 x 10³ cells/50 µl per well and cultured with TC-100 medium (Nosan Corporation) containing 10% FBS (Sigma) in a 96-well plate. The recombinant virus stock solution was diluted to 10⁻⁶, 10⁻⁷, 10⁻⁸, 10⁻⁹, 10⁻¹⁰ (5 serial dilutions) with TC-100 medium containing 10% FBS and the virus was infected by adding 50 µl of each dilution of the virus stock solution to one well of each plate. The plates were sealed to prevent drying and statically cultured at 25°C. The selection of recombinant virus was performed by confirming that a polyhedrin was not formed on day 7 after infection under microscope. And the cellulose binding activity of the recombinant cohesin secreted in the culture medium was analyzed by SDS-PAGE and silver staining, and clones showing activity were selected as a recombinant cohesin-expressing recombinant virus clone (Cj1-3, Cj2-3) (Fig. 2).

### (3) Preparation of recombinant cohesin protein

The recombinant virus was inoculated to the Sf9 cells (Invitrogen) cultured in the EXCELL 420 medium (JRH Bioscience) at an M.O.I of 5 and the supernatant of the culture medium on day 4 after inoculation was used as a recombinant cohesin sample.

### (4) Cellulose binding assay

The cellulose binding assay was performed as follows. Cellulose CBinD 100 Resin (100 mg) (Novagen) was added to 1 ml of the medium in which cohesin had been expressed, and stirred for 60 minutes at room temperature. After stirring, the CBinD 100 Resin was collected by centrifugation at 6,000 rpm for 10 minutes at 4°C. The supernatant was discarded, 1 ml of the washing buffer (Washing buffer: Novagen) was added and the precipitate was suspended. After stirring for 10 minutes at room temperature, it was further centrifuged at 6,000 rpm for 10 minutes at 4°C and the CBinD 100 Resin was collected. This procedure was further repeated twice and the CBinD 100 Resin was washed. Ethylene glycol (100%, 100 µl) was added to the CBinD 100 Resin collected by centrifugation and after suspension, it was left for 10 minutes at room temperature. The supernatant (80 µl) was collected after centrifugation at 6,000 rpm for 10 minutes at 4°C and used as an eluted fraction. The sample for electrophoresis was prepared by adding 40 µl of the SDS-PAGE sample buffer (Laemmli, Nature, vol. 227, pp680-685 (1970)) and boiling for 5 minutes, and SDS-PAGE was performed.

### Example 4

### Acquisition of dockerin gene:

The dockerin gene (sequence Dockerin) was amplified by PCR under the conditions mentioned below by using the vector (given by Associate Professor Sakka, Laboratory of Applied Microbiology, Faculty of Bioscience, Mie University), which was obtained by cloning the dockerin gene of the C. josui CelB (DDBJ/EMBL/Genbank Database accession number D16670.1) into pET-28a(+) vector as a template.

### (1) Design of PCR primers

The nucleotide sequences and the combinations of primers used for the amplification of the dockerin gene were as follows.

Primers for addition of an N-terminal dockerin
DONT 1: (for addition of a restriction enzyme SacI recognition sequence) 5'- ggcgagctcatgggtttaaaaggcgatct -3'
DONT 2: (for addition of a restriction enzyme EcoRI recognition sequence)

5'- ccgaattcattcagcagtttaactt -3'

Primers for addition of a C-terminal dockerin
DOCT 1: (for addition of a restriction enzyme NcoI and Rco81I recognition sequences) 5'- gccatggcctcaggatgggtttaaaaggcgatct -3'
DOCT 2: (for addition of a restriction enzyme NheI recognition sequence)

5'- ggctagcttaattcagcagtttaactt -3'

### (2) PCR conditions

Step 1: 94.0°C 2 minutes
Step 2: 94.0°C 60 seconds
Step 3: 42.0°C 90 seconds
Step 4: 72.0°C 60 seconds
Step 5: Repeat Steps 2 to 4 for 30 times
Step 6: 72.0°C 3 minutes

As a result of the amplification by PCR, a C. Josui CelB dockerin gene fragment of about 200 bp added with restriction enzyme recognition sequences on both ends was obtained.

### Example 5

Construction of transfer vector for addition of C-terminal dockerin:

### (1) Preparation of dockerin gene

Restriction enzymes NcoI (8 to 24U) and NheI (8 to 24U) (both from Takara Shuzo Co., Ltd.) were added to 20 µl of the C. josui CelB dockerin gene PCR product, which had been added with restriction enzyme NcoI and Eco81I recognition sequences at 5' end and a restriction enzyme NheI recognition sequence at 3' end. The restriction enzyme digestion was performed and the end sequences were exposed. Then, the restriction enzyme-treated dockerin gene fragment was purified by using a Qiagen spin column (QIAquick: Qiagen).

### (2) Insertion into transfer vector

After digesting the restriction enzyme recognition sites in the multi-cloning site of transfer vector pYNG for recombinant baculoviral preparation (Katakura Industries Co., Ltd.) with NcoI and XbaI (both from Takara Shuzo Co., Ltd.), the digested ends were dephosphorylated with alkaline phosphatase (Takara Shuzo Co., Ltd.). Solution I (5 µl) of Ligation Kit (Takara Shuzo Co., Ltd.) was added to 0.1 µg (2 µl) of the linearized transfer vector and 0.1 µg (3 µl) of the restriction enzyme-treated dockerin gene fragment. After the reaction at 16°C for 1 hour, *E*. *coli* JM109 (Toyobo Co., Ltd.) was transformed with the whole reaction solution. Ampicillin resistant transformants were selected and plasmids were purified.

Then, several clones were selected and, after the reaction using DNA Sequence Kit (Applied Biosystems Inc.) with yng. for mentioned above as a primer was performed, the nucleotide sequence was analyzed by using an ABI Genetic Analyzer (Applied Biosystems Inc.). A clone inserted the dockerin gene fragment without mutations was referred to as pYNG/CT-DOCK vector.

### (3) Introduction of enterokinase recognition site and multicloning site.

The oligonucleotides having an enterokinase recognition site and a multicloning site were synthesized.
MCS-EK-DOCT+: 
5'- catgggatatcgctagcgaaaacgacgatgacgataaggga
ggcggttcttcacaacagggcc -3'
 MCS-EK-DOCT-:

5'- tgaggccctgttgtgaagaaccgcctcccttatcgtcatcg

tcgttttcgctagcgatatcc -3'

The solution containing 1 µM of each oligonucleotide and 50 mM sodium chloride was prepared and the two oligonucleotides were hybridized by treating at 94°C for 5 minutes, 55°C for 15 minutes and 37°C for 15 minutes (hereinafter referred to as 'EK-DOCK'). Then, Solution I (4 µl) of Ligation Kit (Takara Shuzo Co., Ltd.) was added to 0.1 µg (3 µl) of the linearized transfer vector obtained by treating pYNG/CT-DOCK with restriction enzymes NcoI and Eco81I and 1 µl of the EK-DOCK fragment and reacted at 16°C for 1 hour. Then, *E*. *coli* JM109 (Toyobo Co., Ltd.) was transformed with the whole reaction solution and plasmids were purified from the obtained ampicillin resistant transformants. Several clones were selected and, after the reaction using DNA Sequence Kit (Applied Biosystems Inc.) with yng.for mentioned above as a primer was performed, the nucleotide sequence was analyzed by using an ABI Genetic Analyzer (Applied Biosystems Inc.). A clone that was confirmed to have the nucleotide sequences of the enterokinase recognition site and the multicloning site (BglII, SacI, SmaI, EcoRI, NcoI, EcoRV, and NheI) was referred to as pYNG/EK-CT-DOCK vector for addition of dockerin (Fig. 3).

### Example 6

### Construction of transfer vector for addition of N-terminal dockerin

### (1) Preparation of dockerin gene

Restriction enzymes SacI (8 to 24U) and EcoRI (8 to 24U) (both from Takara Shuzo Co., Ltd.) were added to 20 µl of the C. josui CelB dockerin gene PCR product, which had been added with a restriction enzyme SacI recognition sequence at 5' end and a restriction enzyme EcoRI recognition sequence at 3' end. The restriction enzyme digestion was performed and the end sequences were exposed. Then, the restriction enzyme-treated dockerin gene fragment was purified by using a Qiagen spin column (QIAquick: Qiagen).

### (2) Insertion into transfer vector

After digesting the restriction enzyme recognition sites in the multi-cloning site of transfer vector pYNG30Ksig for recombinant baculoviral preparation (Katakura Industries Co., Ltd.) with SacI and EcoRI (both from Takara Shuzo Co., Ltd.) to make it linear, the digested ends were dephosphorylated with alkaline phosphatase (Takara Shuzo Co., Ltd.). Solution I (5 µl) of Ligation Kit (Takara Shuzo Co., Ltd.) was added to 0.1 µg (2 µl) of the linearized transfer vector and 0.1 µg (3 µl) of the restriction enzyme-treated dockerin gene fragment. After the reaction at 16°C for 1 hour, *E*. *coli* JM109 (Toyobo Co., Ltd.) was transformed with the whole reaction solution. Ampicillin resistant transformants were selected and plasmids were purified.

Then, several clones were selected and, after the reaction using DNA Sequence Kit (Applied Biosystems Inc.) with yng.for mentioned above as a primer was performed, the nucleotide sequence was analyzed by using an ABI Genetic Analyzer (Applied Biosystems Inc.). A clone inserted the dockerin gene fragment without mutations was referred to as pYNG/NT-DOCK vector.

### (3) Introduction of enterokinase recognition site and multicloning site.

The oligonucleotides having an enterokinase recognition site and a multicloning site were synthesized.
MCS-EK-DONT+: 
5'- aattgggaggcggatcagaatcgcagggcgatgacgacgat
aagatctcccgggaatccatggt -3'
 MCS-EK-DONT-:

5'- ctagaccatggaattcccgggtgatcttatcgtcgtcatcg
ccctgcgattctgatccgcctccc -3'

The solution containing 1 µM of each oligonucleotide and 50 mM sodium chloride was prepared and the two oligonucleotides were hybridized by treating at 94°C for 5 minutes, 55°C for 15 minutes and 37°C for 15 minutes (EK-DONT). Then, Solution I (4 µl) of Ligation Kit (Takara Shuzo Co., Ltd.) was added to 0.1 µg (3 µl) of the linearized transfer vector obtained by treating pYNG/NT-DOCK with restriction enzymes EcoRI and XbaI and 1 µl of the EK-DONT fragment and reacted at 16°C for 1 hour. Then, *E. coli* JM109 was transformed with the whole reaction solution and plasmids were purified from the obtained ampicillin resistant transformants.

Then, several clones were selected and, after the reaction using DNA Sequence Kit (Applied Biosystems Inc.) with yng.for mentioned above as a primer was performed, the nucleotide sequence was analyzed by using an ABI Genetic Analyzer (Applied Biosystems Inc.). A clone that was confirmed to have the nucleotide sequences of enterokinase recognition sequence and the multicloning site (BglII, SmaI, EcoRI, NcoI and XbaI) was referred to as pYNG/EK-NT-DOCK vector for addition of dockerin (Fig. 4).

### Example 7

### Expression of green fluorescent protein (GFP) fused with C-terminal dockerin

### (1) Design of PCR primers

Using vector pEGFP-N1 (Clontech) harboring the GFP gene as a template, introduction of restriction enzyme recognition sequences and deletion of the termination codon were performed by PCR. For the preparation of the GFP gene fragment to be inserted into pYNG/EK-CT-DOCK, the following primers were synthesized.
yng.for:

5'- aaccatctcgcaaataaata -3'

Primer 5: (for addition of a restriction enzyme EcoRV recognition sequence)

5'- gggatatctttgtatagttcatcca -3'

### (2) Preparation of PCR sample

| | |
|---|---|
| pEGFP-N1 | 100 pg |
| 10 x Ex-Taq buffer (Takara Shuzo Co., Ltd.) | 5 µl |
| yng.for | 1 ng |
| Primer 5 | 1 ng |
| dNTP mix (Takara Shuzo Co., Ltd.) | 4 µl |
| Ex-Taq (Takara Shuzo Co., Ltd.) | 0.5 µl |

Distilled water Added to make the total volume 50 µl

### (3) PCR conditions

The same operation was performed as in the acquisition of the cohesin gene in Example 1.

### (4) Preparation of GFP gene

Restriction enzymes BglII (8 to 24U) and EcoRV (8 to 24U) (both from Takara Shuzo Co., Ltd.) were added to 20 µl of the GFP gene PCR product, which had been added with a restriction enzyme BglII recognition sequence at 5' end and a restriction enzyme EcoRV recognition sequence at 3' end. The restriction enzyme digestion was performed and the end sequences were exposed. Then, the restriction enzyme-treated GFP gene fragment was purified by using a Qiagen spin column (QIAquick: Qiagen).

### (5) Insertion into transfer vector

After digesting the restriction enzyme recognition sites in the multi-cloning site of transfer vector pYNG/EK-CT-DOCK for addition of a C-terminal dockerin with BglII and EcoRV (both from Takara Shuzo Co., Ltd.) to make it linear, the digested ends were dephosphorylated with alkaline phosphatase (Takara Shuzo Co., Ltd.). Solution I (3 µl) of Ligation Kit (Takara Shuzo Co., Ltd.) was added to 0.1 µg (2 µl) of the linearized transfer vector and 0.1 µg (2 µl) of the restriction enzyme-treated GFP gene fragment. After the reaction at 16°C for 1 hour, *E. coli* JM109 (Toyobo Co., Ltd.) was transformed with the whole reaction solution. Ampicillin resistant transformants were selected and plasmids were purified.

Then, several clones were selected and it was confirmed that the GFP gene fragment was inserted, that the reading frame was correct and that the GFP gene had no mutations. The above-mentioned yng.for and yng.rev primers were used for nucleotide sequence confirmation. After the primer was reacted using DNA Sequence Kit (Applied Biosystems Inc.), the nucleotide sequences were analyzed by using an ABI Genetic Analyzer (Applied Biosystems Inc.).

### Example 8

### Expression of mouse interferon (mIFN) fused with C-terminal dockerin

### (1) Design of PCR primers

Using the mIFN gene (Taniguchi, T et al., J. Biol. Chem., vol. 258, pp9522-9529 (1983); given by Professor Munekawa, Kyoto Institute of Technology) as a template, the introduction of restriction enzyme recognition sequences and deletion of the termination codon were performed by PCR. For the preparation of mIFN gene fragment to be inserted into pYNG/EK-CT-DOCK, the following primers were synthesized.

### Primer 6: (for addition of a restriction enzyme EcoRI recognition sequence)

5'- ccgaattcatgaacaacaggtggatcc -3'

### Primer 7: (for addition of a restriction enzyme EcoRV recognition sequence)

5'- gggatatcgttttggaagtttctggtaa -3'

### (2) Preparation of PCR sample

| | |
|---|---|
| mIFN gene | 100 pg |
| 10 x Ex-Taq buffer (Takara Shuzo Co., Ltd.) | 5 µl |
| Primer 6 | 1 ng |
| Primer 7 | 1 ng |
| dNTP mix (Takara Shuzo Co., Ltd.) | 4 µl |
| Ex-Taq (Takara Shuzo Co., Ltd.) | 0.5 µl |

Distilled water Added to make the total volume 50 µl

### (3) PCR conditions

The same operation was performed as in the acquisition of the cohesin gene.

### (4) Preparation of mIFN gene

Restriction enzymes EcoRI (8 to 24U) and EcoRV (8 to 24U) (both from Takara Shuzo Co., Ltd.) were added to 20 µl of the mIFN gene PCR product, which had been added with a restriction enzyme EcoRI recognition sequence at 5' end and a restriction enzyme EcoRV recognition sequence at 3' end. The restriction enzyme digestionwas performed and the end sequences were exposed. Then, the restriction enzyme-treated mIFN gene fragment was purified by using a Qiagen spin column (QIAquick: Qiagen).

### (5) Insertion into transfer vector

After digesting the restriction enzyme recognition sites in the multi-cloning site of transfer vector pYNG/EK-CT-DOCK for addition of a C-terminal dockerin with EcoRI and EcoRV (both from Takara Shuzo Co., Ltd.) to make it linear, the digested ends were dephosphorylated with alkaline phosphatase (Takara Shuzo Co., Ltd.). Solution I (3 µl) of Ligation Kit (Takara Shuzo Co., Ltd.) was added to 0.1 µg (2 µl) of the linearized transfer vector and 0.1 µg (2 µl) of the restriction enzyme-treated mIFN gene fragment. After the reaction at 16°C for 1 hour, *E*. *coli* JM109 (Toyobo Co., Ltd.) was transformed with the whole reaction solution. Ampicillin resistant transformants were selected and plasmids were purified.

Then, several clones were selected and it was confirmed that the mIFN gene fragment was inserted, that the reading frame was correct and that the mIFN gene had no mutations. The above-mentioned yng.for and yng.rev primers were used for nucleotide sequence confirmation. After the reaction using DNA Sequence Kit (Applied Biosystems Inc.), the nucleotide sequences were analyzed by using an ABI Genetic Analyzer (Applied Biosystems Inc.).

### Example 9

### Expression of mouse interferon (mIFN) fused with N-terminal dockerin

### (1) Design of PCR primers

Using the mIFN gene (given by Professor Munekawa, Kyoto Institute of Technology) as a template, the introduction of restriction enzyme recognition sequences was performed by PCR. For the preparation of the mIFN gene fragment to be inserted into pYNG/EK-NT-DOCK, the following primers were synthesized.

### Primer 8: (for addition of a restriction enzyme EcoRI recognition sequence)

5'- ccgaattcatcaactataagcagctcc -3'

### Primer 9: (for addition of a restriction enzyme XbaI recognition sequence)

5'- ggtctagatcagttttggaagtttctg -3'

### (2) Preparation of PCR sample

| | |
|---|---|
| mIFN gene | 100 pg |
| 10 x Ex-Taq buffer (Takara Shuzo Co., Ltd.) | 5 µl |
| Primer 8 | 1 ng |
| Primer 9 | 1 ng |
| dNTP mix (Takara Shuzo Co., Ltd.) | 4 µl |
| Ex-Taq (Takara Shuzo Co., Ltd.) | 0.5 µl |

Distilled water Added to make the total volume 50 µl

### (3) PCR conditions

The same operation was performed as in the acquisition of the cohesin gene.

### (4) Preparation of mIFN gene

Restriction enzymes EcoRI (8 to 24U) and XbaI (8 to 24U) (both from Takara Shuzo Co., Ltd.) were added to 20 µl of the mIFN gene PCR product, which had been added with a restriction enzyme EcoRI recognition sequence at 5' end and a restriction enzyme XbaI recognition sequence at 3' end. The restriction enzyme digestion was performed and the end sequences were exposed. Then, restriction enzyme-treated mIFN gene fragment was purified by using a Qiagen spin column (QIAquick: Qiagen).

### (5) Insertion into transfer vector

After digesting the restriction enzyme recognition sites in the multi-cloning site of transfer vector pYNG/EK-NT-DOCK for addition of an N-terminal dockerin with EcoRI and XbaI (both from Takara Shuzo Co., Ltd.) to make it linear, the digested ends were dephosphorylated with alkaline phosphatase (Takara Shuzo Co., Ltd.). Solution I (3 µl) of Ligation Kit (Takara Shuzo Co., Ltd.) was added to 0.1 µg (2 µl) of the linearized transfer vector and 0.1 µg (2 µl) of the restriction enzyme-treated mIFN gene fragment. After the reaction at 16°C for 1 hour, *E*. *coli* JM109 (Toyobo Co., Ltd.) was transformed with the whole reaction solution. Ampicillin resistant transformants were selected and plasmids were purified.

Then, several clones were selected and it was confirmed that the mIFN gene fragment was inserted, that the reading frame was correct and that the mIFN gene had no mutations. The above-mentioned yng.for and yng.rev primers were used for nucleotide sequence confirmation. After the reaction using DNA Sequence Kit (Applied Biosystems Inc.), the nucleotide sequences were analyzed by using an ABI Genetic Analyzer (Applied Biosystems Inc.).

### Example 10

### Preparation of recombinant viruses expressing GFP and mIFN fused with C-terminal dockerin

### (1) Cotransfection

Firstly, using a 35 mm petri dish for cell culture, the Bm-N cells were prepared in a confluent monolayer of about 1 x 10⁶ cells in static culture. The linearized ABv DNA (0.1 µg) (Katakura Industries Co., Ltd.) and either of 0.5 µg of the transfer vector of GFP gene fused with the C-terminal dockerin pYNG/EK-CT-DOC/GFP, the transfer vector of mIFN gene fused with the C-terminal dockerin pYNG/EK-CT-DOC/mIFN, or the transfer vector of mIFN gene fused with the N-terminal dockerin pYNG/EK-NT-DOC/mIFN were mixed with 100 µl of TC-100 (Nosan Corporation) in a 1.5 ml tube and left for 15 minutes at room temperature. The cationic lipid reagent (Lipofectin: GIBCO-BRL) (5 µl) was added to 100 µl of TC-100 and left for 15 minutes at room temperature. Then, after these two solutions were mixed and left for an additional 15 minutes at room temperature, 800 µl of TC-100 was added. This mixture was added to the Bm-N cells prepared in a confluent monolayer on the 35 mm petri dish for cell culture. After the cultivation for 20 hours at 25°C, the DNA solution was removed, 2 ml of TC-100 medium containing 10% FBS (Sigma) was added, then it was statically cultured for 7 days at 25°C. The supernatant of this culture medium was used as a recombinant virus stock solution.

### (2) Screening

The isolation of ABv from the obtained virus stock solution as above was performed according to the method reported by King et al. (King, L. A and Possee, R. D., A Laboratory Guide London: Chapman and Hall (1992)). First, the Bm-N cells were adjusted to 1 x 10³ cells/50 µl per well and cultured with TC-100 medium (Nosan Corporation) containing 10% FBS (Sigma) in a 96-well plate. The recombinant virus stock solution was diluted to 10⁻⁶, 10⁻⁷, 10⁻⁸, 10⁻⁹, 10⁻¹⁰ (5 serial dilutions) with TC-100 medium containing 10% FBS and the virus was infected by adding 50 µl of each dilution of the virus stock solution to one well of each plate. The plates were sealed to prevent drying and statically cultured at 25°C. The selection of recombinant virus was performed by confirming that a polyhedrin was not formed on day 7 after infection under microscope.

### (3) Preparation of recombinant protein fused with dockerin

The recombinant virus prepared as above was inoculated to the Sf9 cells cultured in the EXCELL 420 medium (JRH Bioscience) at an M.O.I of 5. The supernatant of the culture medium on day 4 after inoculation was used as a sample of recombinant protein fused with the dockerin.

### Example 11

Detection of recombinant protein fusedwith dockerin (1):

### (1) Detection of GFP by SDS-PAGE and antibody

The detection of GFP was performed by Western blot analysis using an anti-GFP antibody (Boehringer Mannheim) after separating the above samples by SDS-PAGE according to a conventional method.

### (2) Results

As shown in Fig. 5, in the Sf9 cells (Invitrogen) inoculated with the recombinant virus of GFP gene fused with the C-terminal dockerin, GFP was observed in the supernatant and, further, it could be confirmed that this GFP was expressed in a soluble form.

### Example 12

Detection of sample of recombinant protein fused with dockerin (2):

### (1) Detection of mIFN by anti-virus activity

The detection of mIFN activity was performed using the mouse L929 cells as follows. Firstly, after the subculture cells in the logarithmic growth phase were counted using an erythrocytometer, they were prepared at the cell density of 4 to 6 x 10⁶ cells/ml with MEM medium containing 5% of FBS (Sigma) and 100 µl of this solution was added to each well of a 96-well plate. After 24 hours, samples were diluted to 10⁻⁴ to 10⁻⁵ with the medium and further diluted 2-fold using a transfer plate and transferred by overlaying the transfer plate on the culture plate. This was cultured in an incubator at 5% of CO₂ concentration for 12 to 24 hours at 37°C. After the cultivation, mouse encephalomyocarditis virus was diluted to be 1000 to 3000 PFU/ml with the medium and, further, the medium in the culture plate was discarded and the virus solution was added to the wells of columns 1 to 10 at 100 µl/well. The medium without virus was added to the wells of columns 11 and 12 at 100 µl/well. After the cultivation for 24 to 36 hours, the degree of cell degeneration was examined under microscope. When the rate of cell degeneration of the virus-infected control group reached almost 100%, the medium was discarded and the virus was inactivated by UV irradiation for 5 minutes. Then, the cells were immersed in 10% formalin solution for 10 minutes and fixed.

After the fixation, the formalin solution was discarded and the naphthol blue black staining solution was added with 50 µl/well and the staining was performed for 30 minutes. Then, the staining solution was discarded and washed out well with tap water. After the plate was air dried, the stained cells were eluted with 100 mM sodium hydroxide. The eluted plate was measured for absorbance at 600 nm using a microplate reader (Bio-Rad) and titers of the samples were obtained by comparing with the reference. The international standard mIFN from the US NIH was used as a reference.

### (2) Results

Activities of 9.22 x 10⁶ unit/ml in the supernatant of the culture medium inoculated with the recombinant virus of mIFN gene fused with the C-terminal dockerin and 3.51 x 10⁷ unit/ml in the supernatant of the culture medium inoculated with the recombinant virus of mIFN gene fused with the N-terminal dockerin were detected. Therefore, it could be confirmed that mIFN was expressed in a state of retaining its activity.

### Example 13

Purification of recombinant GFP fused with C-terminal dockerin:

### (1) Preparation of column and elution of GFP fused with C-terminal dockerin

First, the preparation of the column having the recombinant cohesin adsorbed thereto was performed as follows. That is, 1 ml of the recombinant cohesin sample prepared in Example 3 was added to 50 mg of cellulose binging support resin (CBinD 100 Resin: Novagen) and mixed for 1 hour at room temperature using a rotating shaker. Then, the mixture of the CBinD 100 Resin and the recombinant cohesin sample was transferred to an Econocolumn (1.0 cm in diameter, 5 cm in length: Bio-Rad) and the CBinD 100 Resin having the recombinant cohesin adsorbed thereto was precipitated. After the CBinD 100 Resin having the recombinant cohesin adsorbed thereto and the supernatant of the culture medium were separated into two phases, the cock in the bottom of the Econocolumn was opened and the supernatant of the culture was eluted. When the surface of the supernatant in the Econocolumn reached the upper surface of the CBinD 100 Resin having the recombinant cohesin adsorbed thereto, 20 ml of 50 mM Tris-HCl (pH 7.2) (hereinafter referred to as 'Washing Buffer') was applied from the top of the column and the CBinD 100 Resin having the recombinant cohesin adsorbed thereto was washed.

The preparation of sample for applying to the above-mentioned column was performed by adding 1 M calcium chloride aqueous solution to the sample of GFP fused with the C-terminal dockerin prepared in Example 10 to be at the final concentration of 10 mM of calcium chloride and mixing them. Then, 5 ml of the sample for applying to the column was applied to the column in which the CBinD 100 Resin having the recombinant cohesin adsorbed thereto was packed. After the sample was applied, the column was washed by adding 20 ml of Washing Buffer thereto. After the column was washed, GFP fused with the C-terminal dockerin was eluted by applying 30 ml of 50 mM Tris-HCl (pH 7.2) containing 50 mM EDTA (hereinafter referred to as 'Elution Buffer').

After the elution, 2 ml of ethylene glycol was applied to the column and the CBinD 100 Resin having the recombinant cohesin protein adsorbed thereto was suspended, and then the suspension was collected to a microtube. After the suspension was mixed for 1 hour at room temperature using a rotation shaker, it was centrifuged and the supernatant was sampled. The sampling was performed at each step of the above-mentioned examples, and the analyses were performed by silver staining of SDS-PAGE and Western blotting using an anti-GFP antibody (Boehringer Mannheim) (Fig. 6).

### (2) Detection of GFP fused with C-terminal dockerin by silver staining of SDS-PAGE and Western blot analysis using anti-GFP antibody:

The samples fractionated in each purification step in Example 13 (1) were separated by SDS-PAGE (Laemmli, Nature, vol.227, pp680-685 (1970)). A SDS-PAGE gel of 12.5% was used and electrophoresed under constant current (20 mA) with the molecular weight markers. After the electrophoresis was over, the protein was confirmed by using a silver staining kit (Ginsen Kit Wako; Wako Pure Chemical Industries, Ltd.) and the purity at each purification step was confirmed. In the analysis by the Western blotting method, after separated by SDS-PAGE, the protein was transferred onto PVDF membrane (Advantec) from the gel by using a blotting apparatus (ATTO Corporation). After the transfer, blocking treatment of the PVDF membrane having the protein adsorbed thereto was performed with T-TBS containing 3% BSA (Wako Pure Chemical Industries, Ltd.). Then, Western blotting was performed by using an anti-GFP mouse antibody (Boehringer Mannheim) as a primary antibody and an anti-mouse IgG rabbit antibody (Wako Pure Chemical Industries, Ltd.) as a secondary antibody. Signal detection was performed by exposure of X-ray film (Fuji Photo Film Co., Ltd.) using ECL reagent (Amersham Biosciences). The measurement of the signal intensity obtained on the X-ray film in the above step was performed by inputting the image to a computer as an image data, and analyzing and quantifying the input data by using NIH image ver. 1.55 software. Thereby the expression amount of recombinant GFP was obtained.

### (3) Results

In lane 11 in Fig. 6, GFP fused with the C-terminal dockerin was confirmed in the sample of GFP fused with the C-terminal dockerin. More specifically, from the results of Western blot analysis, when the samples of GFP fused with the C-terminal dockerin were added to the CBinD 100 Resin having the recombinant cohesin adsorbed thereto, the band was not detected in lanes 4 and 5 (the fractions eluted with Washing Buffer) in Fig. 6, but it was observed in the fraction added with Elution Buffer (lane 6). Also, the corresponding band was observed at the same position from the results of silver staining. The results of the silver stained gels were input to a computer as an image data, and analysis was performed by using NIH image ver. 1.55 software, which revealed that the refined purity of GFP fused with the C-terminal dockerin reached 90% by single column operation.

### Example 14

### Construction of transfer vector for expressing cohesin containing six cohesin domains (Cj6):

After digesting pYNG/Cj1 constructed in Example 2 by SacI and EcoRI (both from Takara Shuzo Co., Ltd.), the digested ends were dephosphorylated with alkaline phosphatase (Takara Shuzo Co., Ltd.). Subsequently, from pMK-2C Cj CipA vector, which was the same as used in Example 1, a gene fragment containing six cohesin domains was obtained by digestion with SacI and EcoRI (both from Takara Shuzo Co., Ltd.). Solution I (5 µl) of Ligation Kit (Takara Shuzo Co., Ltd.) was added to 0.1 µg (3 µl) of the gene fragment and 0.1 µg (2 µl) of the linearized pYNG/Cj1. After the reaction at 16°C for 1 hour, *E*. *coli* JM109 (Toyobo Co., Ltd.) was transformed with the whole reaction solution. Ampicillin resistant transformants were selected and plasmids were purified.

Then the above-mentioned yng.for and yng. rev primers were used for nucleotide sequence confirmation of several clones in the same manner as Example 2. After the reaction of the primers using DNA Sequence Kit (Applied Biosystems Inc.), the nucleotide sequences were analyzed by using an ABI Genetic Analyzer (Applied Biosystems Inc.). A clone in which insertion of six cohesin domains was confirmed was referred to as pYNG/Cj6 (Fig. 7).

### Example 15

### Preparation of cohesin-expressingrecombinant virus (2):

Cotransfection and screening were performed according to the method of Example 3, except that pYNG/Cj6 prepared as above was used as a transfer vector, and the clones showing the cellulose binding activity were selected as a recombinant cohesin-expressing recombinant virus clone (Cj6-1).

### Example 16

### Preparation of recombinant cohesin protein

### (1) Production of recombinant cohesin protein by silkworm larvae

The recombinant viruses prepared in Examples 3 and 15 (Cj1-3, Cj2-3 and Cj6-1) were inoculated subcutaneously to the day-1 silkworm larvae of the fifth instar. The larvae were raised with artificial feed (Morus: Katakura Industries Co., Ltd.) under the conditions of 45% humidity and at 25°C. Then the body fluid was collected 6 days after inoculation. The collected body fluid was centrifuged at 12, 500g for 60 minutes, then the supernatant was collected to remove contaminated tissue fragments or the like. The thus obtained supernatant was diluted 10-fold with 50 mM potassium phosphate buffer (pH 7.0; hereinafter referred to as 'Buffer A'), thereby the body fluid containing the recombinant cohesin protein was prepared.

### (2) Purification of recombinant cohesin protein expressed in body fluid of silkworm

Q sepharose anion exchanger (Q sepharose Fast Flow; Amersham Biosciences) was equilibrated with Buffer A, then it was transferred to an Econocolumn (2.5 cm in diameter, 5 cm in length: Bio-Rad), and the body fluid containing the recombinant cohesin protein was added thereto to have it adsorbed. Subsequently, after it was washed with Buffer A containing 0.1 M sodium chloride, the recombinant cohesin protein was eluted by increasing the salt concentration to 0.5 M. The fraction containing the thus obtained recombinant cohesin protein was referred to as the purified recombinant cohesin protein fraction.

### (3) Detection of cohesin protein (cohesin domain)

After the samples containing the purified recombinant cohesin protein were separated by SDS-PAGE, the separated protein was transferred onto PVDF membrane (Advantec) from the gel by using a blotting apparatus (ATTO Corporation). After the transfer, blocking treatment of the PVDF membrane having the protein adsorbed thereto was performed with 50 mM Tris-HCl buffer containing 5% skim milk (hereinafter referred to as 'Blocking Buffer 1'). The PVDF membrane after blocking treatment was treated with 100-fold dilution of the samples of the recombinant protein fused with the dockerin prepared in Example 10 (supernatant of the culture medium of Sf9 cells containing GFP fused with the C-terminal dockerin) with Blocking Buffer 1 at room temperature for 2 hours. With the PVDF membrane after the treatment, Western blot analysis was performed by using an anti-GFP mouse antibody (Boehringer Mannheim) as a primary antibody and an anti-mouse IgG rabbit antibody (Wako Pure Chemical Industries, Ltd.) as a secondary antibody. Signal detection was performed by exposure of X-ray film (Fuji Photo Film Co., Ltd.) using ECL reagent (Amersham Biosciences).

### (4) Results

Almost no nonspecific reaction was observed by detection using GFP fused with the C-terminal dockerin, and all the recombinant cohesin proteins having one, two and six cohesin domains could be detected (Fig. 8).

### Example 17

### Expression of green fluorescent protein (GFP) fused with N-terminal dockerin tag

### (1) Design of PCR primers

Using vector pEGFP-N1 (Clontech) harboring the GFP gene as a template, introduction of restriction enzyme recognition sequences and deletion of the termination codon were performed by PCR. For the preparation of the GFP gene fragment to be inserted into pYNG/EK-NT-DOCK, the following primers were synthesized.

Primer 4: (for addition of a restriction enzyme BglII recognition sequence): 5'- ggagatctccatgagtaaaggagaagaa -3' yng.rev

5'- cgcacagaatctaacgctta -3'

### (2) Preparation of PCR sample

| | |
|---|---|
| pEGFP-N1 | 100 pg |
| 10 x Ex-Taq buffer (Takara Shuzo Co., Ltd.) | 5 µl |
| Primer 4 | 1 ng |
| primer yng.rev | 1 ng |
| dNTP mix (Takara Shuzo Co., Ltd.) | 4 µl |
| Ex-Taq (Takara Shuzo Co., Ltd.) | 0.5 µl |

Distilled water Added to make the total volume 50 µl

### (3) PCR conditions

The same operation was performed as in the acquisition of the cohesin gene in Example 1.

### (4) Preparation of GFP gene

Restriction enzymes BglII (8 to 24U) and EcoRV (8 to 24U) (both from Takara Shuzo Co., Ltd.) were added to 20 µl of the GFP gene PCR product, which had been added with a restriction enzyme BglII recognition sequence at 5' end and a restriction enzyme EcoRV recognition sequence at 3' end. Restriction enzyme digestion was performed and the end sequences were exposed. Then, the restriction enzyme-treated GFP gene fragment was purified by using a Qiagen spin column (QIAquick: Qiagen).

### (5) Insertion into transfer vector

After digesting the restriction enzyme recognition sites in the multi-cloning site of transfer vector pYNG/EK-NT-DOCK for addition of an N-terminal dockerin with BglII and EcoRV (both from Takara Shuzo Co., Ltd.) to make it linear, the digested ends were dephosphorylated with alkaline phosphatase (Takara Shuzo Co., Ltd.). Solution I (3 µl) of Ligation Kit (Takara Shuzo Co., Ltd.) was added to 0.1 µg (2 µl) of the linearized transfer vector and 0.1 µg (2 µl) of the restriction enzyme-treated GFP gene fragment. After the reaction at 16°C for 1 hour, *E*. *coli* JM109 (Toyobo Co., Ltd.) was transformed with the whole reaction solution. Ampicillin resistant transformants were selected and plasmids were purified.

Then, several clones were selected and it was confirmed that the GFP gene fragment was inserted, that the reading frame was correct and that the GFP gene had no mutations. The above-mentioned yng.for and yng.rev primers were used for nucleotide sequence confirmation. After the primer was reacted using DNA Sequence Kit (Applied Biosystems Inc.), the nucleotide sequences were analyzed by using an ABI Genetic Analyzer (Applied Biosystems Inc.).

### Example 18

### Preparation of recombinant virus expressing GFP fused with N-terminal dockerin:

Cotransfection and screening were performed according to the same method as Example 3, and a recombinant virus expressing GFP fused with an N-terminal dockerin was prepared. Except that the transfer vector prepared in Example 17 was used as a transfer vector.

### Example 19

### Immobilization of cohesin protein (cohesin domain) on support

### (1) Preparation of support having cohesin protein (cohesin domain) immobilized thereon.

A coupling resin (6 g) for ligand immobilization (CNBr-activated Sepharose 4 Fast Flow; Amersham Biosciences) was swollen in 2 L of 1 mM HCl, then washed. The coupling support for ligand immobilization was divided equally into three portions of 5.3 g (wet weight). Then, 14 mg (protein mass) of each fraction containing the recombinant cohesin protein having one, two or six cohesin domains purified in Example 16 was added thereto, and coupling was performed at room temperature for 2 hours. After the coupling was completed, blocking treatment of the support was performed with 1 M aminoethanol at room temperature for 2 hours. Subsequently, the resin was washed alternately with 100 mM Tris-HCl buffer (pH 8.0) containing 0.5 M sodium chloride and 100 mM acetic acid containing 0.5 M sodium chloride for three times each. The support having the cohesin domains immobilized thereon by covalent bond (hereinafter, the resin having one cohesin domain is referred to as 'Cj1 resin', the resin having two cohesin domains as 'Cj2 resin', and the resin having six cohesin domains as 'Cj6 'rersin) was equilibrated with 50 mM Tris-HCl buffer containing 500 mM sodium chloride (pH 7.2; hereinafter referred to as 'Buffer B'), then stored in Buffer B containing 10 mM calcium chloride.

### (2) Measurement of protein concentration

The concentrations of the proteins covalently bound to the Cj1 resin, Cj2 resin and Cj6 resin, which were prepared as above, were determined by measuring absorbance according to a conventional method, using bovine serum albumin as a standard protein solution and Micro BCA Protein Assay Kit (Pierce). The absorbance was measured at 550 nm by using Microplate Reader Model 450 (Bio-Rad).

### (3) Results

The protein concentrations of the above-mentioned supports were 14 mg, 14 mg and 11 mg respectively. Therefore, the support wherein the cohesin protein having one, two or six cohesin domains had been immobilized on the coupling resin for ligand immobilization by covalent bond could be prepared.

### Example 20

Purification of protein fused with dockerin with resin having cohesin protein (cohesin domain) immobilized thereon by covalent bond

### (1) Preparation of protein fused with dockerin

The recombinant virus expressing GFP fused with the C-terminal dockerin tag, which was prepared in Example 10 was inoculated subcutaneously to the day-1 silkworm larvae of the fifth instar. The larvae were raised with artificial feed (Morus: Katakura Industries Co., Ltd.) under the conditions of 45% humidity and at 25°C. Then the body fluid was collected 6 days after inoculation. The collected body fluid was centrifuged at 12,500g for 60 minutes, then the supernatant was collected to remove contaminated tissue fragments or the like. The thus obtained supernatant was diluted 10-fold with Buffer B containing 10 mM calcium chloride, and thereby the body fluid containing the recombinant protein fused with the dockerin was prepared.

### (2) Purification of protein fused with dockerin

The supports having the cohesin protein (cohesin domain) immobilized thereon by covalent bond, which was prepared in Example 19 (Cj1 resin, Cj2 resin and Cj6 resin) were packed respectively in the Econocolumns (Bio-Rad) with a diameter of 1.5 cm up to the height of 4.0 cm. After the Econocolumn Flow Adaptor was attached thereto, 170 ml of the body fluid containing the recombinant protein fused with the dockerin prepared in the above-mentioned (1) was applied to each column. After the resins were washed with the same volume of Buffer B, they were eluted with Buffer B containing 50 mM EDTA.

Detection of GFP was performed by analysis of silver staining or Western blot with an anti-GFP antibody (Boehringer Mannheim) after the samples were separated by SDS-PAGE according to a conventional method.

### (4) Results

In all the Cj1 resin, Cj2 resin and Cj6 resin, adsorption of GFP fused with the C-terminal dockerin tag and elution with Buffer B containing 50 mM EDTA could be confirmed. In a case of Cj1 support, the purity of GFP fused with the C-terminal dockerin tag, which was 3.1% in the body fluid of larvae, increased to 85.7% after the purification operation (Fig. 9).

### Example 21

Cleavage of protein fused with dockerin by enterokinase.

### (1) Preparation of protein fused with dockerin

The recombinant virus expressing GFP fused with the C-terminal or N-terminal dockerin tag prepared in Examples 10 or 18 was inoculated subcutaneously to the silkworm pupae 1 day after pupation. The pupae were stored under the conditions of 45% humidity and at 25°C and collected 7 days after inoculation. The 10 ml of sodium phosphate buffer (pH 7.4) containing 10% glycerol was added per 2 pupae, which was treated with a homogenizer (HG30: Hitachi Ltd.) for 1 minute, and with an ultrasonic homogenizer (USP-600: Shimadzu Corporation) for 2 minutes. After the mixture was centrifuged at low speed of 3, 000 rpm for 10 minutes, the obtained supernatant was filtered. Thereby homogenized pupae solution containing the protein fused with the dockerin was prepared.

### (2) Purification of protein fused with dockerin

The resin (Cj2 resin) (100 µl) having the cohesin domain immobilized thereon by covalent bond prepared in Example 19 and 1, 150 µl of Buffer B were added to 250 µl of the homogenized pupae solution, and the mixture was rotated at 4°C for 2 hours by using a revolution mixer RVM-100 (Iwaki Co., Ltd.) to have it adsorbed. The obtained Cj2 resin was washed three times by centrifugation with 1 ml of Buffer B for each washing, then eluted with 200 µl of Buffer B containing 50 mM EDTA or 2 mM EGTA at 4°C. In addition, Buffer B was added to the remaining resin to obtain another eluate, which was combined to the eluate in the previous step.

### (3) Cleavage of tag by enterokinase

To 700 µl of the obtained eluate, 100 unit of enterokinase (Biozyme Laboratories, Ltd.) was added, which was incubated at 4°C for 48 hours. The confirmation of cleavage of the tag with enterokinase was performed by analysis of silver staining or Western blot with an anti-GFP antibody (Boehringer Mannheim) after the samples were separated by SDS-PAGE according to a conventional method.

### (4) Results

Similar elution effects were observed with 2 mM EGTA and 50 mM EDTA. In addition, both GFP fused with the N-terminal dockerin tag and GFP fused with the C-terminal dockerin tag were cleaved by enterokinase (Fig. 10).

### Example 22

### Labeling of cohesin protein by biotinylation

### (1) Biotinylation of cohesin protein

The fraction containing the cohesin protein with one cohesin domain, which was purified in Example 16, was concentrated to 1 mg/ml of protein mass by using Centriprep YM-10 (Millipore). Biotinylation of the cohesin protein was performed by using ECL protein biotinylation system (Amersham Biosciences). More specifically, the solvent of 2.5 ml of the concentrated cohesin protein solution was replaced with sodium hydrogen carbonate buffer, subsequently biotinylation reagent was added thereto according to a conventional method, and then the mixture was shaken at room temperature for 1 hour. Thereafter, themixturewas added to SephadexG25 column included in Kit, then the fraction containing biotinylated cohesin protein having one cohesin domain was obtained by flowing PBS.

### (2) Detection of recombinant protein fused with dockerin by biotinylated cohesin

The homogenized pupae solution and the purified eluate of GFP fused with the C-terminal or N-terminal dockerin tag was prepared according to the same method as Example 21. The recombinant virus expressing mIFN fused with the C-terminal or N-terminal dockerin tag, which was prepared in Example 10 were inoculated, collected, prepared according to the same method as Example 21, and the homogenized pupae solution was obtained. The homogenized pupae solutions and the purified eluate were separated by SDS-PAGE according to a conventional method, and then the protein was silver stained, transferred onto PVDF membrane (Advantec) from the gel by using or blotting apparatus (ATTO Corporation). After the transfer, blocking treatment of the PVDF membrane having the protein adsorbed thereto was performed with Tris buffer containing 1% skim milk and 0.05% Tween 20 (hereinafter referred to as 'Blocking Buffer 2'). The PVDF membrane after the blocking treatment was treated in the 1,000-fold dilution of the fraction containing the biotinylated cohesin with Blocking Buffer 2 at room temperature for 1 hour. The PVDF membrane treated with the biotinylated cohesin was analyzed by Western blotting using streptavidin (Antigenix America Inc.) labeled with horseradish peroxidase. Signal detection was performed by developing color with DAB (Wako Pure Chemical Industries, Ltd.) and hydrogen peroxide.

### (3) Results

By using the biotinylated cohesin, GFP fused with the C-terminal or N-terminal dockerin tag and mIFN fused with the C-terminal or N-terminal dockerin tag could be specifically detected at high sensitivity (Fig. 11).

### Industrial Applicability

In the purification method of the present invention, a recombinant fused protein can be separated and purified with the use of specific binding of dockerin and cohesin regardless of the properties of target proteins by producing the recombinant fused protein wherein the target protein has been fused with dockerin by genetic engineering techniques.

Accordingly, the purification method of the present invention enables to separate and purify a recombinant fused protein wherein a given target protein has been fused with dockerin. Particularly, if are combinant fused protein in which a chemical or enzymatic cleavage site has been inserted between the target protein and the dockerin is used, only the target protein can be easily purified and separated. Therefore, the purification method of the present invention is extremely advantageous as a protein purification and separation method by genetic engineering.

## Claims

1. A method of purifying a recombinant fused protein **characterized in that** a recombinant fused protein, wherein a target protein has been fused with dockerin, is treated with a support having a cohesin domain immobilized thereon.

2. The purification method according to claim 1, wherein the recombinant fused protein is treated to be adsorbed to the support having the cohesin domain immobilized thereon with calcium ion, and subsequently the calcium is eliminated by treating with a metal chelate.

3. The purification method according to claim 1 or 2, wherein the recombinant fused protein has a chemical or enzymatic cleavage site between the target protein and the dockerin.

4. The purification method according to claim 3, wherein the enzymatic cleavage site is an enterokinase cleavage site (DDDDK).

5. The purification method according to claim 1, wherein an immobilization method makes use of a hydrogen bond or a covalent bond.

6. An expression vector into which a gene encoding the target protein and a gene encoding the dockerin have been inserted to produce the recombinant fused protein used in the method according to claim 1.

7. The expression vector according to claim 6, wherein a gene encoding a chemical or enzymatic cleavage site has been inserted between the gene encoding the target protein and the gene encoding the dockerin.

8. The expression vector according to claim 6 or 7 derived from baculovirus.

9. A method of producing a target protein **characterized by** comprising obtaining a recombinant fused protein having a target protein bound to dockerin with the use of an expression vector having genes encoding the target protein and the dockerin inserted thereinto, subsequently purifying the recombinant fused protein by binding to a support having a cohesin domain immobilized thereon, and then eliminating the dockerin from the recombinant fused protein.

10. The method of producing a target protein according to claim 9, wherein the expression vector in which a gene encoding a chemical or enzymatic cleavage site has been inserted between a gene encoding the target protein and a gene encoding the dockerin.

11. A method of detecting and quantifying a recombinant fused protein **characterized by** binding a recombinant fused protein, wherein a target protein has been fused with dockerin, to a labeled cohesin domain, and then measuring the labeled amount after eliminating the non-binding labeled cohesin domain from the measurement system.

12. The detection and quantification method according to claim 11, wherein the labeling is biotinylation.

13. A method of detecting or quantifying a cohesin domain **characterized by** binding a cohesin domain to a labeled dockerin, and then measuring the labeled amount after eliminating the non-binding labeled dockerin.

14. A method of purifying a cohesin domain **characterized in that** a solution containing a cohesin domain is treated by adsorption with an anion exchange support to have the cohesin domain adsorbed to the anion exchange support, and subsequently the cohesin domain is eluted by increasing salt concentration.


SEQUENCE LISTING

<110> KATAKURA INDUSTRIES CO., LTD.

<120> METHOD FOR PURIFICATION OF RECOMBINANT HYBRID PROTEIN
      AND METHOD FOR PRODUCTION OF PROTEIN USING THE SAME

<130> PF-020018-WO

<140>
<141>

<150> JP 2001-314079
<151> 2001-10-11

<160> 23

<170> PatentIn Ver. 2.1

<210> 1
<211> 1303
<212> DNA
<213> Artificial Sequence

<220>
<223> Inventor: NOMURA, Tsuyoshi; NAGAYA, Hidekazu;
      SHIMAMURA, Akio; NAYA, Shinichi; KANAYA,
      Toshimichi; SAKKA, Kazuo; OHMIYA, Kunio

<220>
<223> Description of Artificial Sequence:Cj1

<400> 1
ccggatccat gcgtaaaaag tctttagcat ttttgctagc actaacaatg ttggtgacat 60
tattaggagc tcagcttaca gcttttgcag ctgatactgg cgtcatatca gttcaattta 120
ataatggtag ttcaccaaca tcatcaagtt caatatacgc tagatttaaa gttacaaaca 180

caagtggttc accaatcaac ttggcagatt tgaaacttag atattatttt actcaggatg 240
aaaataagca aatgacattc tggtgtgacc atgcaggtta tctgagtggt aacaactata 300
tggatgttac ttcaaaggta tctggaacat ttaatgaagt aagccctgca gttacaaatg 360
cagatcatta tcttgaagtt gcattaagca gtgatgcagg aagtcttcca gctggaggat 420
ctatagaaat tcaaaccaga tttgcaagaa acgactggtc taattttgat caatcaaatg 480
actggtcata tacttcagct ggttcataca tggattggca gaaaattgct gctttcgtag 540
gcggaactct tgtatatggt tcaacaccta atggtgatga caacccaaca caagacccaa 600
aaatcagtcc tacttctatt tctgcaaaac agggacaatt ctcagacgct gtaatagctc 660
ttacaccaaa tggaaataca tttaatggaa ttactgagtt gcagagtaac caatatgtaa 720
agggaacaaa ccaagtaaca atattggcta gctatttgaa tacattgcca gcaaatagca 780
caaaaactct tacatttgat ttcggtgtag gttcaaagaa tcctaaattg actatcaatg 840
taggtgaaag tggtaataca aatggtctta aggtttcagt aggaacagct gttggtgctc 900
ctggtgatac agtaacagtt cctgttacat ttgctgatgt agcaaaagta aacaacgtag 960
gaacatgtaa cttctatctt ggatatgatg caagtctttt ggatgtagta tcagtagatg 1020
caggtccaat agttaagaat gcagcagtaa acttctcaag cagtgcaagc aacggaacaa 1080
tcagcttcct gttcttggac aacacaatca ctgatgaatt gattacttca gatggtgtgt 1140
tcgcaaatat cacatttaag attaagagta ctgctacaca aggtacaaca ccaataacct 1200
tcaaagatgg aggagctttt ggtgacggta ctatgtcaaa gatagcttca gttattaaga 1260
caagtggtag tgtagttata agtccagatc cttagtctag acc 1303

<210> 2
<211> 1747
<212> DNA
<213> Artificial Sequence

<220>
<223> Description of Artificial Sequence:Cj2

<400> 2
ccggatccat gcgtaaaaag tctttagcat ttttgctagc actaacaatg ttggtgacat 60
tattaggagc tcagcttaca gcttttgcag ctgatactgg cgtcatatca gttcaattta 120
ataatggtag ttcaccaaca tcatcaagtt caatatacgc tagatttaaa gttacaaaca 180
caagtggttc accaatcaac ttggcagatt tgaaacttag atattatttt actcaggatg 240
aaaataagca aatgacattc tggtgtgacc atgcaggtta tctgagtggt aacaactata 300
tggatgttac ttcaaaggta tctggaacat ttaatgaagt aagccctgca gttacaaatg 360

cagatcatta tcttgaagtt gcattaagca gtgatgcagg aagtcttcca gctggaggat 420
ctatagaaat tcaaaccaga tttgcaagaa acgactggtc taattttgat caatcaaatg 480
actggtcata tacttcagct ggttcataca tggattggca gaaaattgct gctttcgtag 540
gcggaactct tgtatatggt tcaacaccta atggtgatga caacccaaca caagacccaa 600
aaatcagtcc tacttctatt tctgcaaaac agggacaatt ctcagacgct gtaatagctc 660
ttacaccaaa tggaaataca tttaatggaa ttactgagtt gcagagtaac caatatgtaa 720
agggaacaaa ccaagtaaca atattggcta gctatttgaa tacattgcca gcaaatagca 780
caaaaactct tacatttgat ttcggtgtag gttcaaagaa tcctaaattg actatcaatg 840
taggtgaaag tggtaataca aatggtctta aggtttcagt aggaacagct gttggtgctc 900
ctggtgatac agtaacagtt cctgttacat ttgctgatgt agcaaaagta aacaacgtag 960
gaacatgtaa cttctatctt ggatatgatg caagtctttt ggatgtagta tcagtagatg 1020
caggtccaat agttaagaat gcagcagtaa acttctcaag cagtgcaagc aacggaacaa 1080
tcagcttcct gttcttggac aacacaatca ctgatgaatt gattacttca gatggtgtgt 1140
tcgcaaatat cacatttaag attaagagta ctgctacaca aggtacaaca ccaataacct 1200
tcaaagatgg aggagctttt ggtgacggta ctatgtcaaa gatagcttca gttattaaga 1260
caagtggtag tgtagttata agtccagatc ctacaaatgc tcttaaagta acagtaggaa 1320
cagcagaagg taatgttgga gaaacagtaa cagttcctgt tacatttgct gatgtagcaa 1380
aagtaaacaa cgtaggaaca tgtaacttct atcttgcata tgatgcaagt cttttggatg 1440
tagtatcagt agatgcaggt ccaatagtta agaatgcagc agtaaacttc tcaagcagtg 1500
caagcaacgg aacaatcagc ttcctgttct tggataacac aatcactgac gaattgatta 1560
cttcagatgg tgtgtttgca aatatcacat tcaaattaaa gaatgtatca actaaaacaa 1620
caacaccaat aaccttcaaa gacggaggag catttggtga cggtactatg tcaaagataa 1680
ctacagttat caagacaaac ggtagtgtaa caattattcc tggtgaccca gaaccttagt 1740
ctagacc 1747

<210> 3
<211> 3489
<212> DNA
<213> Artificial Sequence

<220>
<223> Description of Artificial Sequence:Cj6

<400> 3
atgcgtaaaa agtctttagc atttttgcta gcactaacaa tgttggtgac attattagga 60

gctcagctta cagcttttgc agctgatact ggcgtcatat cagttcaatt taataatggt 120
agttcaccaa catcatcaag ttcaatatac gctagattta aagttacaaa cacaagtggt 180
tcaccaatca acttggcaga tttgaaactt agatattatt ttactcagga tgaaaataag 240
caaatgacat tctggtgtga ccatgcaggt tatctgagtg gtaacaacta tatggatgtt 300
acttcaaagg tatctggaac atttaatgaa gtaagccctg cagttacaaa tgcagatcat 360
tatcttgaag ttgcattaag cagtgatgca ggaagtcttc cagctggagg atctatagaa 420
attcaaacca gatttgcaag aaacgactgg tctaattttg atcaatcaaa tgactggtca 480
tatacttcag ctggttcata catggattgg cagaaaattg ctgctttcgt aggcggaact 540
cttgtatatg gttcaacacc taatggtgat gacaacccaa cacaagaccc aaaaatcagt 600
cctacttcta tttctgcaaa acagggacaa ttctcagacg ctgtaatagc tcttacacca 660
aatggaaata catttaatgg aattactgag ttgcagagta accaatatgt aaagggaaca 720
aaccaagtaa caatattggc tagctatttg aatacattgc cagcaaatag cacaaaaact 780
cttacatttg atttcggtgt aggttcaaag aatcctaaat tgactatcaa tgtaggtgaa 840
agtggtaata caaatggtct taaggtttca gtaggaacag ctgttggtgc tcctggtgat 900
acagtaacag ttcctgttac atttgctgat gtagcaaaag taaacaacgt aggaacatgt 960
aacttctatc ttggatatga tgcaagtctt ttggatgtag tatcagtaga tgcaggtcca 1020
atagttaaga atgcagcagt aaacttctca agcagtgcaa gcaacggaac aatcagcttc 1080
ctgttcttgg acaacacaat cactgatgaa ttgattactt cagatggtgt gttcgcaaat 1140
atcacattta agattaagag tactgctaca caaggtacaa caccaataac cttcaaagat 1200
ggaggagctt ttggtgacgg tactatgtca aagatagctt cagttattaa gacaagtggt 1260
agtgtagtta taagtccaga tcctacaaat gctcttaaag taacagtagg aacagcagaa 1320
ggtaatgttg gagaaacagt aacagttcct gttacatttg ctgatgtagc aaaagtaaac 1380
aacgtaggaa catgtaactt ctatcttgca tatgatgcaa gtcttttgga tgtagtatca 1440
gtagatgcag gtccaatagt taagaatgca gcagtaaact tctcaagcag tgcaagcaac 1500
ggaacaatca gcttcctgtt cttggataac acaatcactg acgaattgat tacttcagat 1560
ggtgtgtttg caaatatcac attcaaatta aagaatgtat caactaaaac aacaacacca 1620
ataaccttca aagacggagg agcatttggt gacggtacta tgtcaaagat aactacagtt 1680
atcaagacaa acggtagtgt aacaattatt cctggtgacc cagaacctac agaagatctt 1740
aacgtagcag taggaacagc agaaggtaat gttggagaaa cagtaacagt tcctgtaaca 1800
tttgctaatg tagcaaaagt aaacaacgta ggaacatgta acttctatct tgcatatgat 1860
gcaagtcttt tggatgtagt atcagtagat gcaggtccaa tagttaagaa tgcagcagta 1920
aacttctcaa gcagtgcaag caatggaaca atcagcttcc tgttcttaga taacacaatc 1980
actgatgaat tgattacttc agatggtgtg tttgcaaata tcacattcaa attaaagaat 2040
gtatcaacta aaacaacaac accaataagc ttcaaagatg gaggagcatt tggtgacggt 2100
aatatggcaa agatagctac agttgttaaa acaaacggta gtgtaacaat tattcctggt 2160
gatccagaac ctacagaaga tcttaacgta gcagtaggaa cagcagaagg taatgttgga 2220

gatacagtaa cagttcctgt aacatttgct aacgtagcaa aagtaaacaa cgtaggaaca 2280
tgtaacttct atcttacata tgatgcaagt cttttggatg tagtatcagt agatgcaggt 2340
ccaatagtta agaatgcagc agtaaacttc tcaagcagtg caagcaacga aacaatcagc 2400
ttcttgttct tagataacac aatcactgac gaattgatta cttcagatgg tgtgtttgca 2460
aatatcacat tcaaattaaa gagtgtatca actaaaacaa caacaccaat aagcttcaaa 2520
gacggaggag catttggtga cggtactatg gcaaagatag ctacagttgt taagacaaat 2580
ggtagtgtaa caattattcc tggtgatcca gaacctacag aagatcttaa tgtagcagta 2640
ggaacagcag aaggtaatgt tggagataca gtaacagttc ctgtaacatt tgctaacgta 2700
gcaaaagtaa acaacatagg aacatgtaac ttctatctta catatgatgc aagcttgtta 2760
gatgtagtat cagtagctgc aggtccaata gttaagaatg cagcagtaaa cttctcaagc 2820
agtgcaagca acggaacaat cagcttcttg ttcttagata acacaatcac tgacgaattg 2880
attacttcag atggtgtgtt tgcaaatatc acattcaaat taaagagtgt atcaactaaa 2940
acaacaacac caataagctt caaagacgga ggagcatttg gtgacggtac tatggcaaag 3000
atagctacag ttgttaagac aaatggtagt gttactatcg atgttggtga gcctgttgtt 3060
acaggacttg gagtaaagat tgcttcagta acaggaaaaa ctggtgatac tataacagta 3120
cctgtaactc tcagcaatgt tgctacagta ggtaatgtag gaacatgtaa tttctatatt 3180
acatatgacc caaccttgtt gcaggctgta tcagcaacag ctggtgatat agtaataaat 3240
gcacctgtta acttctcaag cagtatcaat gcaacaaatg gtactatcag tattcttttc 3300
cttgataaca ctataactga tcaacctatc gcaagtgatg gagtaatcac taaccttact 3360
ttcaaagtat taggttcttc aagcacaact actcctatcg ctttcaaagc aggtggagca 3420
ttcggtaatg gtaacatggc aaaaatcagt gatatcacat tcacaaatgg aagtgcaaaa 3480
cttaattaa 3489

<210> 4
<211> 183
<212> DNA
<213> Clostridium josui

<220>
<223> dockerin

<400> 4
atgggtttaa aaggcgatgt caataatgat ggtgctatag atgcccttga tattgctgcg 60
ctcaagaagg ctattttgac tcaatcaact tctaatatta atttaacaaa tgctgatatg 120
aataatgacg gaaatattga tgccattgat tttgctcagc taaaagttaa actgctgaat 180

taa 183

<210> 5
<211> 34
<212> DNA
<213> Artificial Sequence

<220>
<223> Description of Artificial Sequence:DOCT1

<400> 5
gccatggcct caggatgggt ttaaaaggcg atct 34

<210> 6
<211> 27
<212> DNA
<213> Artificial Sequence

<220>
<223> Description of Artificial Sequence:DOCT2

<400> 6
ggctagctta attcagcagt ttaactt 27

<210> 7
<211> 29
<212> DNA
<213> Artificial Sequence

<220>
<223> Description of Artificial Sequence:DONT1

<400> 7

ggcgagctca tgggtttaaa aggcgatct                                    29

<210> 8
<211> 25
<212> DNA
<213> Artificial Sequence

<220>
<223> Description of Artificial Sequence:DONT2

<400> 8
ccgaattcat tcagcagttt aactt                                        25

<210> 9
<211> 63
<212> DNA
<213> Artificial Sequence

<220>
<223> Description of Artificial Sequence:MCS-EK-DOCT+

<400> 9
catgggatat cgctagcgaa aacgacgatg acgataaggg aggcggttct tcacaacagg 60
gcc                                                                63

<210> 10
<211> 62
<212> DNA
<213> Artificial Sequence

<220>
<223> Description of Artificial Sequence:MCS-EK-DOCT-

<400> 10
tgaggccctg ttgtgaagaa ccgcctccct tatcgtcatc gtcgttttcg ctagcgatat 60
cc 62

<210> 11
<211> 64
<212> DNA
<213> Artificial Sequence

<220>
<223> Description of Artificial Sequence:MCS-EK-DONT+

<400> 11
aattgggagg cggatcagaa tcgcagggcg atgacgacga taagatctcc cgggaatcca 60
tggt 64

<210> 12
<211> 65
<212> DNA
<213> Artificial Sequence

<220>
<223> Description of Artificial Sequence:MCS-EK-DONT-

<400> 12
ctagaccatg gaattcccgg gtgatcttat cgtcgtcatc gccctgcgat tctgatccgc 60
ctccc 65

<210> 13
<211> 28
<212> DNA
<213> Artificial Sequence

<220>
<223> Description of Artificial Sequence:primer1

<400> 13
ccggatccat gcgtaaaaag tctttagc 28

<210> 14
<211> 29
<212> DNA
<213> Artificial Sequence

<220>
<223> Description of Artificial Sequence:primer2

<400> 14
ggtctagact aaggatctgg acttataac 29

<210> 15
<211> 29
<212> DNA
<213> Artificial Sequence

<220>
<223> Description of Artificial Sequence:primer3

<400> 15
ggtctagact aaggttctgg gtcaccagg 29

<210> 16
<211> 28
<212> DNA
<213> Artificial Sequence

<220>
<223> Description of Artificial Sequence:primer4

<400> 16
ggagatctcc atgagtaaag gagaagaa                                          28

<210> 17
<211> 25
<212> DNA
<213> Artificial Sequence

<220>
<223> Description of Artificial Sequence:primer5

<400> 17
gggatatctt tgtatagttc atcca                                             25

<210> 18
<211> 27
<212> DNA
<213> Artificial Sequence

<220>
<223> Description of Artificial Sequence:primer6

<400> 18
ccgaattcat gaacaacagg tggatcc                                           27

<210> 19
<211> 28
<212> DNA
<213> Artificial Sequence

<220>
<223> Description of Artificial Sequence:primer7

<400> 19
gggatatcgt tttggaagtt tctggtaa 28

<210> 20
<211> 27
<212> DNA
<213> Artificial Sequence

<220>
<223> Description of Artificial Sequence:primer8

<400> 20
ccgaattcat caactataag cagctcc 27

<210> 21
<211> 27
<212> DNA
<213> Artificial Sequence

<220>
<223> Description of Artificial Sequence:primer9

<400> 21
ggtctagatc agttttggaa gtttctg 27

<210> 22
<211> 20
<212> DNA
<213> Artificial Sequence

<220>
<223> Description of Artificial Sequence:yng.for

<400> 22
aaccatctcg caaataaata                                                    20

<210> 23
<211> 20
<212> DNA
<213> Artificial Sequence

<220>
<223> Description of Artificial Sequence:yng.rev

<400> 23
cgcacagaat ctaacgctta                                                    20